# EUROPEAN PATENT APPLICATION

(11) **EP 3 613 747 A1**
(43) Date of publication of application: **26.02.2020**
(21) Application number: 18190659.5
(22) Date of filing: 24.08.2018
(51) Int. Cl.: C07D 491/052

(54) **CRYSTALLINE FORMS OF TRANS-6'-FLUORO-N-METHYL-4-PHENYL-4',9'-DIHYDRO-3'H-SPIRO[CYCLOHEXANE-1,1'-PYRANO[3,4-B]INDOL]-4-AMINE**

(71) Applicant: Grünenthal GmbH, 52078 Aachen (DE)
(72) Inventor: The designation of the inventor has not yet been filed

(57) **Abstract**

The present invention relates to a crystalline form of the compound according to formula (I) 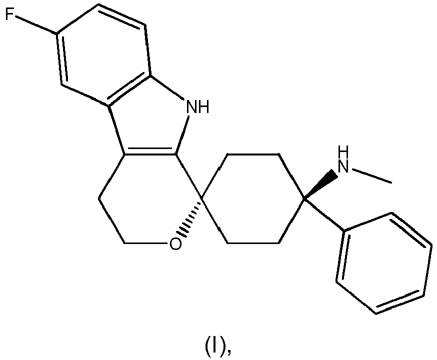 having at least one X-ray powder diffraction peak (CuK_{α} radiation) in the range of from 9.3±0.2 to 11.3±0.2 (2θ) and/or 16.0±0.2 to 18.0±0.2 (2θ) and/or 17.0±0.2 to 19.5±0.2 (2θ) and/or 18.5±0.2 to 21.0±0.2 (2θ).

## Description

### FIELD OF THE PRESENT INVENTION

The present invention relates to a crystalline form of the compound according to formula (I) 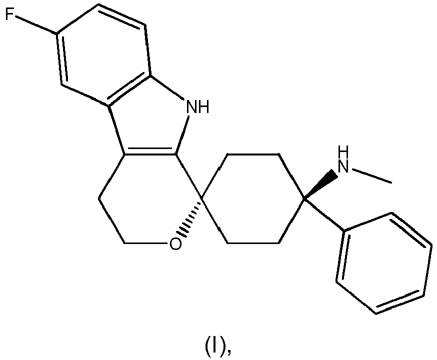 having at least one X-ray powder diffraction peak (CuK_{α} radiation) in the range of from 9.3±0.2 to 11.3±0.2 (2θ) and/or 16.0±0.2 to 18.0±0.2 (2θ) and/or 17.0±0.2 to 19.5±0.2 (2θ) and/or 18.5±0.2 to 21.0±0.2 (2θ); as well as pharmaceutical dosage forms comprising at least one crystalline form of the compound according to formula (I), the use of these crystalline forms as well as to processes for producing these crystalline forms.

### BACKGROUND OF THE PRESENT INVENTION

Pharmacologically active ingredients can exist in different solid forms, i.e. different crystalline forms having different physical, physicochemical and chemical properties.

Different physical or physicochemical properties can cause different crystalline forms of the same drug to have largely different processing and storage performance. Such physical or physicochemical properties include, for example, thermodynamic stability, crystal morphology (form, shape, structure, particle size, particle size distribution, color, degree of crystallinity, flowability, density, bulk density, powder density, apparent density, vibrated density, hardness, deformability, grindability, compressability, compactability, brittleness, elasticity), caloric properties (particularly melting point), solubility (particularly equilibrium solubility, pH dependence of solubility), dissolution (particularly dissolution rate, intrinsic dissolution rate), hygroscopicity, tackiness, adhesiveness, tendency to electrostatic charging, and the like.

In addition, different chemical properties can cause different crystalline forms of the same drug to have largely different performance properties. For example, a crystalline form having a low hygroscopicity (relative to other crystalline forms) can have superior chemical stability and longer shelf-life stability (cf. R. Hilfiker, Polymorphism, 2006 Wiley VCH, p. 235-242 and 251-252).

In medicine, the treatment of pain is of great importance and although a significant number of drugs are known for and established in the treatment of pain, there remains, for instance with regard to drug-related side-effects, a demand for improved pain medication, especially for the treatment of strong/severe and/or chronic and/or neuropathic pain. Consequently, a great deal of effort is still being invested by pharmaceutical companies into the development of new, improved analgesics.

One particular drug that is of great interest especially for the use in treating pain, especially acute, neuropathic and/or chronic pain is *trans*-6'-fluoro-*N*-methyl-4-phenyl-4',9'-dihydro-3'*H-*spiro[cyclohexane-1,1'-pyrano[3,4-b]indol]-4-amine which is described in WO 2004/043967 and the chemical structure of which is depicted in formula (I) below:

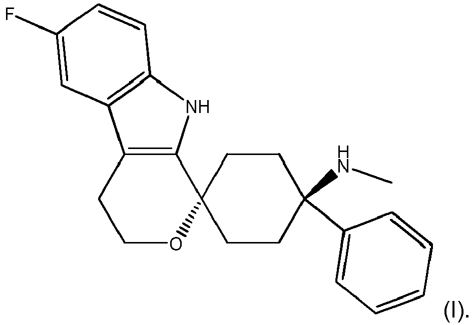

The solid forms of *trans*-6'-fluoro-*N*-methyl-4-phenyl-4',9'-dihydro-3'*H*-spiro[cyclohexane-1,1'-pyrano[3,4-*b*]indol]-4-amine that are known so far are not satisfactory in every respect and consequently there is a demand for advantageous solid forms, especially crystalline forms. In particular, there is a demand for crystalline forms of *trans*-6'-fluoro-*N*-methyl-4-phenyl-4',9'-dihydro-3'*H*-spiro[cyclohexane-1,1'-pyrano[3,4-*b*]indol]-4-amine for use in pharmaceutical dosage forms.

This object has been achieved by the present invention. It has surprisingly been found that different crystalline forms of *trans*-6'-fluoro-*N*-methyl-4-phenyl-4',9'-dihydro-3'*H*-spiro-[cyclohexane-1,1'-pyrano[3,4-b]indol]-4-amine can be prepared which have advantageous properties, especially for the use in pharmaceutical dosage forms. These inventive crystalline forms are described herein.

### BRIEF DESCRIPTION OF THE DRAWINGS

Figures 1 to 11 show the x-ray powder diffraction (XRPD) analyses of crystalline forms A to K.
Figures 12 and 13 show the dynamic vapor sorption (DVS) isotherm plots of crystalline form D and F, respectively.

### DETAILED DESCRIPTION

The present invention relates to a crystalline form of the compound according to formula (I) 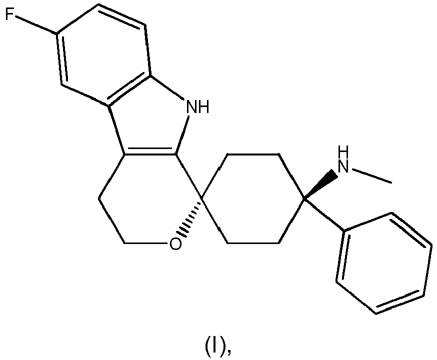 having at least one X-ray powder diffraction peak (CuK_{α} radiation) in the range of from 9.3±0.2 to 11.3±0.2 (2θ) and/or 16.0±0.2 to 18.0±0.2 (2θ) and/or 17.0±0.2 to 19.5±0.2 (2θ) and/or 18.5±0.2 to 21.0±0.2 (2θ).

In a preferred embodiment, the crystalline form according to the present invention has at least one X-ray powder diffraction peak (CuK_{α} radiation) in each of the ranges of from 9.3±0.2 to 11.3±0.2 (2θ), 16.0±0.2 to 18.0±0.2 (2θ), 17.0±0.2 to 19.5±0.2 (2θ) and 18.5±0.2 to 21.0±0.2 (2θ).

In the following, the compound according to formula (I) is also referred to by the term "pharmacologically active ingredient".

The compound according to formula (I) is preferably present in form of the free base. The definition of the term "free base" as used herein shall include solvates, co-crystals and crystalline forms. For the purposes of this specification, "free base" preferably means that the compound according to formula (I) is not present in form of a co-crystal or salt, particularly not in form of an acid-addition salt. The most basic functional groups of the compound according to formula (I) are the two amino moieties, which according to the present invention are preferably neither protonated nor quaternized. In other words, the free electron pairs of the nitrogen atoms of the amino moieties are present as a Lewis base. Methods to determine whether a chemical substance is present as the free base or as a salt are known to the skilled artisan such as ¹⁴N or ¹⁵N solid state NMR, x-ray diffraction, x-ray powder diffraction, IR, Raman, XPS. ¹H-NMR recorded in solution may also be used to consider the presence of protonation.

Preferably, the crystalline form according to the present invention is an ansolvate or a solvate. In a particularly preferred embodiment, the crystalline form according to the present invention is an ansolvate.

In another preferred embodiment, the crystalline form according to the present invention is a solvate, preferably selected from the group of hydrates, solvates of lower alcohols, such as methanol, ethanol, 1-propanol or 2-propanol or solvates of toluene or a solvate of solvate mixtures. More preferably, the crystalline form according to the present invention is a solvate selected from the group consisting of methanol solvate, 2-propanol solvate, ethanol solvate and dimethyl sulfoxide solvate. Preferably, the solvate is selected from the group consisting of monosolvate, hemi-solvate, disolvate, trisolvate, and mixtures thereof. According to this embodiment, the solvate preferably is a variable or non-stoichiometric solvate.

Preferably, the crystalline form according to the present invention is not hygroscopic. The person skilled in the art knows how to determine hygroscopicity. Preferably, hygroscopicity is determined via DVS (dynamic vapor sorption), preferably by using a Porotec DVS at 25°C (cycles: 50-90% relative humidity/90-0% relative humidity/0-90% relative humidity/90-50% relative humidity). Further preferably, the hygroscopicity is classified according to the ranges for mass increase defined in the European Pharmacopoeia: very hygroscopic (vh): increase of the mass ≥ 15 %; hygroscopic (h): increase of the mass is less than 15 % and equal or greater than 2 %; slightly hygroscopic (sh): increase of the mass is less than 2 % and equal or greater than 0.2 %; not hygroscopic (nh): increase of the mass is less than 0.2 %; deliquescent (d): sufficient water is absorbed to form a liquid.

Unless explicitly stated otherwise, all 2θ values refer to an x-ray powder diffraction (XRPD) analysis measured using CuK_{α} radiation having a wavelength of 1.54060 Å. The terms 2θ values and degrees 2θ are used synonymously. A person skilled in the art knows how to realize XRPD analysis. Preferably, XRPD are carried out in transmission geometry with a STOE StadiP or a Panalytical X'Pert Pro X-ray powder diffractometer in reflection geometry with monochromatised CuK_{α} radiation.

According to the present invention, the crystalline form has at least one X-ray powder diffraction peak (CuK_{α} radiation) in the range of from 9.3±0.2 to 11.3±0.2 (2θ) and/or 16.0±0.2 to 18.0±0.2 (2θ) and/or 17.0±0.2 to 19.5±0.2 (2θ) and/or 18.5±0.2 to 21.0±0.2 (2θ). More preferably, the crystalline form has an X-ray powder diffraction peak (CuK_{α} radiation) in the range of from 16.0±0.2 to 18.0±0.2 (2θ). Still more preferably, the crystalline form has X-ray powder diffraction peaks (CuK_{α} radiation) in the range of from 16.0±0.2 to 18.0±0.2 (2θ) and 17.0±0.2 to 19.5±0.2 (2θ). Even more preferably, the crystalline form has X-ray powder diffraction peaks (CuK_{α} radiation) in the range of from 16.0±0.2 to 18.0±0.2 (2θ), 17.0±0.2 to 19.5±0.2 (2θ) and 18.5±0.2 to 21.0±0.2 (2θ). Yet more preferably, the crystalline form has X-ray powder diffraction peaks (CuK_{α} radiation) in the range of from 16.0±0.2 to 18.0±0.2 (2θ), 17.0±0.2 to 19.5±0.2 (2θ), 18.5±0.2 to 21.0±0.2 (2θ) and 9.3±0.2 to 11.3±0.2 (2θ).

In a preferred embodiment, the crystalline form according to the present invention has at least one additional X-ray powder diffraction peak (CuK_{α} radiation) in the range of from 23.7±0.2 to 25.7±0.2 (2θ) and/or 19.9±0.2 to 21.9±0.2 (2θ) and/or 17.4±0.2 to 19.4±0.2 (2θ). More preferably, the crystalline form has an additional X-ray powder diffraction peak (CuK_{α} radiation) in the range of from 23.7±0.2 to 25.7±0.2 (2θ). Still more preferably, the crystalline form has additional X-ray powder diffraction peaks (CuK_{α} radiation) in the range of from 23.7±0.2 to 25.7±0.2 (2θ) and 19.9±0.2 to 21.9±0.2 (2θ). In another preferred embodiment, the crystalline form has additional X-ray powder diffraction peaks (CuK_{α} radiation) in the range of from 23.7±0.2 to 25.7±0.2 (2θ), 19.9±0.2 to 21.9±0.2 (2θ) and 17.4±0.2 to 19.4±0.2 (2θ).

Preferably, the X-ray powder diffraction peaks exhibit a relative intensity of at least 10%, more preferably at least 20%, still more preferably at least 30%, yet more preferably at least 40%, even more preferably at least 50%, most preferably at least 60% or at least 70%, and in particular at least 80% or at least 90% or at least 95%.

In a preferred embodiment, the crystalline form according to the present invention is crystalline form A having at least one X-ray powder diffraction peak (CuK_{α} radiation) selected from the group consisting of 19.5±0.2 (2θ), 14.2±0.2 (2θ), 9.9±0.2 (2θ), 10.3±0.2 (2θ), 17.6±0.2 (2θ), 26.0±0.2 (2θ), 17.2±0.2 (2θ), 15.8±0.2 (2θ) and 22.4±0.2 (2θ); or
crystalline form B having at least one X-ray powder diffraction peak (CuK_{α} radiation) selected from the group consisting of 18.4±0.2 (2θ), 19.1±0.2 (2θ), 7.9±0.2 (2θ), 18.0±0.2 (2θ), 20.0±0.2 (2θ), 12.6±0.2 (2θ), 16.9±0.2 (2θ) and 9.0±0.2 (2θ); or
crystalline form C having at least one X-ray powder diffraction peak CuK_{α} radiation) selected from the group consisting of 18.1±0.2 (2θ), 17.6±0.2 (2θ), 19.1±0.2 (2θ), 8.8±0.2 (2θ), 12.6±0.2 (2θ), 19.5±0.2 (2θ), 14.6±0.2 (2θ), 16.9±0.2 (2θ) and 20.5±0.2 (2θ); or
crystalline form D having at least one X-ray powder diffraction peak (CuK_{α} radiation) selected from the group consisting of 19.4±0.2 (2θ), 12.0±0.2 (2θ), 20.7±0.2 (2θ), 16.5±0.2 (2θ), 11.7±0.2 (2θ), 10.4±0.2 (2θ), 14.5±0.2 (2θ) and 16.8±0.2 (2θ); or
crystalline form E having at least one X-ray powder diffraction peak (CuK_{α} radiation) selected from the group consisting of 10.3±0.2 (2θ), 18.4±0.2 (2θ), 22.6±0.2 (2θ), 20.6±0.2 (2θ), 9.2±0.2 (2θ), 13.8±0.2 (2θ), 17.8±0.2 (2θ), 17.3±0.2 (2θ), 18.9±0.2 (2θ), 23.4±0.2 (2θ), 18.6±0.2 (2θ), 19.4±0.2 (2θ), 21.4±0.2 (2θ), 24.9±0.2 (2θ) and 19.1±0.2 (2θ); or
crystalline form F having at least one X-ray powder diffraction peak (CuK_{α} radiation) selected from the group consisting of 16.9±0.2 (2θ), 16.0±0.2 (2θ), 24.7±0.2 (2θ), 20.9±0.2 (2θ), 18.4±0.2 (2θ), 25.6±0.2 (2θ) and 17.6±0.2 (2θ); or
crystalline form G having at least one X-ray powder diffraction peak (CuK_{α} radiation) selected from the group consisting of 19.1±0.2 (2θ), 18.4±0.2 (2θ), 12.4±0.2 (2θ), 9.5±0.2 (2θ), 18.0±0.2 (2θ), 15.8±0.2 (2θ), 17.2±0.2 (2θ), 29.1±0.2 (2θ) and 20.2±0.2 (2θ); or
crystalline form H having at least one X-ray powder diffraction peak (CuK_{α} radiation) selected from the group consisting of 18.5±0.2 (2θ), 17.5±0.2 (2θ), 19.2±0.2 (2θ), 9.6±0.2 (2θ), 15.1±0.2 (2θ), 22.4±0.2 (2θ), 23.5±0.2 (2θ), 12.5±0.2 (2θ), 19.4±0.2 (2θ) and 16.4±0.2 (2θ); or
crystalline form I having at least one X-ray powder diffraction peak (CuK_{α} radiation) selected from the group consisting of 18.1±0.2 (2θ), 17.6±0.2 (2θ), 19.1±0.2 (2θ), 12.6±0.2 (2θ), 8.8±0.2 (2θ), 19.5±0.2 (2θ), 9.0±0.2 (2θ) and 14.6±0.2 (2θ); or
crystalline form J having at least one X-ray powder diffraction peak (CuK_{α} radiation) selected from the group consisting of 18.5±0.2 (2θ), 19.2±0.2 (2θ), 17.5±0.2 (2θ), 9.6±0.2 (2θ), 15.1±0.2 (2θ), 18.0±0.2 (2θ), 23.6±0.2 (2θ), 12.5±0.2 (2θ) and 19.4±0.2 (2θ); or
crystalline form K having at least one X-ray powder diffraction peak (CuK_{α} radiation) selected from the group consisting of 19.0±0.2 (2θ), 19.6±0.2 (2θ), 19.2±0.2 (2θ), 5.9±0.2 (2θ), 22.0±0.2 (2θ), 23.8±0.2 (2θ), 29.2±0.2 (2θ), 11.5±0.2 (2θ), 37.7±0.2 (2θ) and 29.7±0.2 (2θ).

In a particularly preferred embodiment, the crystalline form according to the present invention is
crystalline form A having X-ray powder diffraction peaks (CuK_{α} radiation) at 19.5±0.2 (2θ), 14.2±0.2 (2θ) and 9.9±0.2 (2θ); or
crystalline form B having X-ray powder diffraction peaks (CuK_{α} radiation) at 18.4±0.2 (2θ), 19.1±0.2 (2θ) and 7.9±0.2 (2θ); or
crystalline form C having X-ray powder diffraction peaks (CuK_{α} radiation) at 18.1±0.2 (2θ), 17.6±0.2 (2θ) and 19.1±0.2 (2θ); or
crystalline form D having X-ray powder diffraction peaks (CuK_{α} radiation) at 19.4±0.2 (2θ), 12.0±0.2 (2θ) and 20.7±0.2 (2θ); or
crystalline form E having X-ray powder diffraction peaks (CuK_{α} radiation) at 10.3±0.2 (2θ), 18.4±0.2 (2θ) and 22.6±0.2 (2θ); or
crystalline form F having X-ray powder diffraction peaks (CuK_{α} radiation) at 16.9±0.2 (2θ), 16.0±0.2 (2θ) and 24.7±0.2 (2θ); or
crystalline form G having X-ray powder diffraction peaks (CuK_{α} radiation) at 19.1±0.2 (2θ), 18.4±0.2 (2θ) and 12.4±0.2 (2θ); or
crystalline form H having X-ray powder diffraction peaks (CuK_{α} radiation) at 18.5±0.2 (2θ), 17.5±0.2 (2θ) and 19.2±0.2 (2θ); or
crystalline form I having X-ray powder diffraction peaks (CuK_{α} radiation) at 18.1±0.2 (2θ), 17.6±0.2 (2θ) and 19.1±0.2 (2θ); or
crystalline form J having X-ray powder diffraction peaks (CuK_{α} radiation) at 18.5±0.2 (2θ), 19.2±0.2 (2θ) and 17.5±0.2 (2θ); or
crystalline form K having X-ray powder diffraction peaks (CuK_{α} radiation) at 19.0±0.2 (2θ), 19.6±0.2 (2θ) and 19.2±0.2 (2θ).

Preferably, the crystalline form according to the present invention is crystalline form F, B, A, C, D, E, G, I, J or K, more preferably F, B or H, still more preferably F or B and most preferably F.

In a particularly preferred embodiment, the crystalline form according to the present invention is crystalline form F. According to this embodiment, the crystalline form preferably has an X-ray powder diffraction peak (CuK_{α} radiation) at 16.9±0.2 (2θ), more preferably the crystalline form has X-ray powder diffraction peaks (CuK_{α} radiation) at 16.9±0.2 (2θ) and 16.0±0.2 (2θ), still more preferably the crystalline form has X-ray powder diffraction peaks (CuK_{α} radiation) at 16.9±0.2 (2θ), 16.0±0.2 (2θ) and 24.7±0.2 (2θ), even more preferably the crystalline form has X-ray powder diffraction peaks (CuK_{α} radiation) at 16.9±0.2 (2θ), 16.0±0.2 (2θ), 24.7±0.2 (2θ) and 20.9±0.2 (2θ), most preferably the crystalline form has X-ray powder diffraction peaks (CuK_{α} radiation) at 16.9±0.2 (2θ), 16.0±0.2 (2θ), 24.7±0.2 (2θ), 20.9±0.2 (2θ) and 18.4±0.2 (2θ), and in particular the crystalline form has X-ray powder diffraction peaks (CuK_{α} radiation) at 16.9±0.2 (2θ), 16.0±0.2 (2θ), 24.7±0.2 (2θ), 20.9±0.2 (2θ), 18.4±0.2 (2θ) and 25.6±0.2 (2θ). Further according to this embodiment, all aforementioned X-ray powder diffraction peaks preferably exhibit a relative intensity of at least 25%.

Preferably, a characteristic X-ray powder diffraction peak (CuK_{α} radiation) of crystalline form F serving to better differentiate crystalline form F from crystalline forms A, B, C, D, E, G, H, I, J and K, 24.7±0.2 (2θ) which preferably exhibits a relative intensity of more than 40%. Therefore, crystalline form F preferably comprises X-ray powder diffraction peaks (CuK_{α} radiation) at 16.9±0.2 (2θ), 16.0±0.2 (2θ) and 24.7±0.2 (2θ), wherein the peak at 24.7±0.2 (2θ) preferably exhibits a relative intensity of more than 40%.

In another preferred embodiment, the crystalline form according to the present invention is crystalline form B. According to this embodiment, the crystalline form preferably has an X-ray powder diffraction peak (CuK_{α} radiation) at 18.4±0.2 (2θ), more preferably the crystalline form has X-ray powder diffraction peaks (CuK_{α} radiation) at 18.4±0.2 (2θ) and 19.1±0.2 (2θ), still more preferably the crystalline form has X-ray powder diffraction peaks (CuK_{α} radiation) at 18.4±0.2 (2θ), 19.1±0.2 (2θ) and 7.9±0.2 (2θ), most preferably the crystalline form has X-ray powder diffraction peaks (CuK_{α} radiation) at 18.4±0.2 (2θ), 19.1±0.2 (2θ), 7.9±0.2 (2θ) and 18.0±0.2 (2θ), and in particular the crystalline form has X-ray powder diffraction peaks (CuK_{α} radiation) at 18.4±0.2 (2θ), 19.1±0.2 (2θ), 7.9±0.2 (2θ), 18.0±0.2 (2θ) and 20.0±0.2 (2θ). Further according to this embodiment, all aforementioned X-ray powder diffraction peaks preferably exhibit a relative intensity of at least 30%.

Preferably, a characteristic X-ray powder diffraction peak (CuK_{α} radiation) of crystalline form B serving to better differentiate crystalline form B from crystalline forms A, C, D, E, F, G, H, I, J and K, in particular from H, I and J, is 7.9±0.2 (2θ) which preferably exhibits a relative intensity of more than 55%. Therefore, crystalline form B preferably comprises X-ray powder diffraction peaks (CuK_{α} radiation) at 18.4±0.2 (2θ), 19.1±0.2 (2θ) and 7.9±0.2 (2θ), wherein the peak at 7.9±0.2 (2θ) preferably exhibits a relative intensity of more than 55%.

In still another preferred embodiment, the crystalline form according to the present invention is crystalline form A. According to this embodiment, the crystalline form preferably has an X-ray powder diffraction peak (CuK_{α} radiation) at 19.5±0.2 (2θ), more preferably the crystalline form has X-ray powder diffraction peaks (CuK_{α} radiation) at 19.5±0.2 (2θ) and 14.2±0.2 (2θ), still more preferably the crystalline form has X-ray powder diffraction peaks (CuK_{α} radiation) at 19.5±0.2 (2θ), 14.2±0.2 (2θ) and 9.9±0.2 (2θ), most preferably the crystalline form has X-ray powder diffraction peaks (CuK_{α} radiation) at 19.5±0.2 (2θ), 14.2±0.2 (2θ), 9.9±0.2 (2θ) and 10.3±0.2 (2θ), and in particular the crystalline form has X-ray powder diffraction peaks (CuK_{α} radiation) at 19.5±0.2 (2θ), 14.2±0.2 (2θ), 9.9±0.2 (2θ), 10.3±0.2 (2θ) and 17.6±0.2 (2θ). Further according to this embodiment, all aforementioned X-ray powder diffraction peaks preferably exhibit a relative intensity of at least 30%.

Preferably, a characteristic X-ray powder diffraction peak (CuK_{α} radiation) of crystalline form A serving to better differentiate crystalline form A from crystalline forms B, C, D, E, F, G, H, I, J and K are 14.2±0.2 (2θ) and 9.9±0.2 (2θ) which preferably both exhibit relative intensities of more than 35%. Therefore, crystalline form A preferably comprises X-ray powder diffraction peaks (CuK_{α} radiation) at 19.5±0.2 (2θ), 14.2±0.2 (2θ) and 9.9±0.2 (2θ), wherein the peaks at 14.2±0.2 (2θ) and 9.9±0.2 (2θ) preferably exhibit relative intensities of more than 35%.

In yet another preferred embodiment, the crystalline form according to the present invention is crystalline form C. According to this embodiment, the crystalline form preferably has an X-ray powder diffraction peak (CuK_{α} radiation) at 18.1±0.2 (2θ), more preferably the crystalline form has X-ray powder diffraction peaks (CuK_{α} radiation) at 18.1±0.2 (2θ) and 17.6±0.2 (2θ), most preferably the crystalline form has X-ray powder diffraction peaks (CuK_{α} radiation) at 18.1±0.2 (2θ), 17.6±0.2 (2θ) and 19.1±0.2 (2θ), and in particular the crystalline form has X-ray powder diffraction peaks (CuK_{α} radiation) at 18.1±0.2 (2θ), 17.6±0.2 (2θ), 19.1±0.2 (2θ) and 8.8±0.2 (2θ). Further according to this embodiment, all aforementioned X-ray powder diffraction peaks preferably exhibit a relative intensity of at least 40%.

Preferably, a characteristic X-ray powder diffraction peak (CuK_{α} radiation) of crystalline form C serving to better differentiate crystalline form C from crystalline forms A, B, D, E, F, G, H, I, J and K, in particular from I, is 17.6±0.2 (2θ) which preferably exhibits a relative intensity of more than 70%. Therefore, crystalline form C preferably comprises X-ray powder diffraction peaks (CuK_{α} radiation) at 18.1±0.2 (2θ), 17.6±0.2 (2θ) and 19.1±0.2 (2θ), wherein the peak at 17.6±0.2 (2θ) preferably exhibits a relative intensity of more than 70%.

In a further preferred embodiment, the crystalline form according to the present invention is crystalline form D. According to this embodiment, the crystalline form preferably has an X-ray powder diffraction peak (CuK_{α} radiation) at 19.4±0.2 (2θ), more preferably the crystalline form has X-ray powder diffraction peaks (CuK_{α} radiation) at 19.4±0.2 (2θ) and 12.0±0.2 (2θ), still more preferably the crystalline form has X-ray powder diffraction peaks (CuK_{α} radiation) at 19.4±0.2 (2θ), 12.0±0.2 (2θ) and 20.7±0.2 (2θ), most preferably the crystalline form has X-ray powder diffraction peaks (CuK_{α} radiation) at 19.4±0.2 (2θ), 12.0±0.2 (2θ), 20.7±0.2 (2θ) and 16.5±0.2 (2θ), and in particular the crystalline form has X-ray powder diffraction peaks (CuK_{α} radiation) at 19.4±0.2 (2θ), 12.0±0.2 (2θ), 20.7±0.2 (2θ), 16.5±0.2 (2θ) and 11.7±0.2 (2θ). Further according to this embodiment, all aforementioned X-ray powder diffraction peaks preferably exhibit a relative intensity of at least 25%.

Preferably, a characteristic X-ray powder diffraction peak (CuK_{α} radiation) of crystalline form D serving to better differentiate crystalline form D from crystalline forms A, B, C, E, F, G, H, I, J and K, in particular from A, is 12.0±0.2 (2θ) which preferably exhibits a relative intensity of more than 85%. Therefore, crystalline form C preferably comprises X-ray powder diffraction peaks (CuK_{α} radiation) at 19.4±0.2 (2θ), 12.0±0.2 (2θ) and 20.7±0.2 (2θ), wherein the peak at 12.0±0.2 (2θ) preferably exhibits a relative intensity of more than 85%.

In still a further preferred embodiment, the crystalline form according to the present invention is crystalline form E. According to this embodiment, the crystalline form preferably has an X-ray powder diffraction peak (CuK_{α} radiation) at 10.3±0.2 (2θ), more preferably the crystalline form has X-ray powder diffraction peaks (CuK_{α} radiation) at 10.3±0.2 (2θ) and 18.4±0.2 (2θ), still more preferably the crystalline form has X-ray powder diffraction peaks (CuK_{α} radiation) at 10.3±0.2 (2θ), 18.4±0.2 (2θ) and 22.6±0.2 (2θ), even more preferably the crystalline form has X-ray powder diffraction peaks (CuK_{α} radiation) at 10.3±0.2 (2θ), 18.4±0.2 (2θ), 22.6±0.2 (2θ) and 20.6±0.2 (2θ), yet more preferably the crystalline form has X-ray powder diffraction peaks (CuK_{α} radiation) at 10.3±0.2 (2θ), 18.4±0.2 (2θ), 22.6±0.2 (2θ), 20.6±0.2 (2θ) and 9.2±0.2 (2θ), most preferably the crystalline form has X-ray powder diffraction peaks (CuK_{α} radiation) at 10.3±0.2 (2θ), 18.4±0.2 (2θ), 22.6±0.2 (2θ), 20.6±0.2 (2θ), 9.2±0.2 (2θ) and 13.8±0.2 (2θ) and in particular the crystalline form has X-ray powder diffraction peaks (CuK_{α} radiation) at 10.3±0.2 (2θ), 18.4±0.2 (2θ), 22.6±0.2 (2θ), 20.6±0.2 (2θ), 9.2±0.2 (2θ), 13.8±0.2 (2θ) and 17.8±0.2 (2θ). Further according to this embodiment, all aforementioned X-ray powder diffraction peaks preferably exhibit a relative intensity of at least 40%.

Preferably, a particularly characteristic X-ray powder diffraction peak (CuK_{α} radiation) of crystalline form E serving to better differentiate crystalline form E from crystalline forms A, B, C, D, F, G, H, I, J and K is 10.3±0.2 (2θ) which preferably exhibits a relative intensity of more than 95%. Therefore, crystalline form E preferably comprises X-ray powder diffraction peaks (CuK_{α} radiation) at 10.3±0.2 (2θ), 18.4±0.2 (2θ) and 22.6±0.2 (2θ), wherein the peak at 10.3±0.2 (2θ) preferably exhibits a relative intensity of more than 95%.

In yet a further preferred embodiment, the crystalline form according to the present invention is crystalline form G. According to this embodiment, the crystalline form preferably has an X-ray powder diffraction peak (CuK_{α} radiation) at 19.1±0.2 (2θ), more preferably the crystalline form has X-ray powder diffraction peaks (CuK_{α} radiation) at 19.1±0.2 (2θ) and 18.4±0.2 (2θ) and most preferably the crystalline form has X-ray powder diffraction peaks (CuK_{α} radiation) at 19.1±0.2 (2θ), 18.4±0.2 (2θ) and 12.4±0.2 (2θ). Further according to this embodiment, both aforementioned X-ray powder diffraction peaks preferably exhibit a relative intensity of at least 9%.

Preferably, characteristic X-ray powder diffraction peaks (CuK_{α} radiation) of crystalline form G serving to better differentiate crystalline form G from crystalline forms A, B, C, D, E, F, H, I, J and K, in particular from K, are 19.1±0.2 (2θ) which preferably exhibits a relative intensity of more than 95% and 18.4±0.2 (2θ) which preferably exhibits a relative intensity of more than 15% Therefore, crystalline form G preferably comprises X-ray powder diffraction peaks (CuK_{α} radiation) at 19.1±0.2 (2θ), 18.4±0.2 (2θ) and 12.4±0.2 (2θ), wherein the peak at 19.1±0.2 (2θ) preferably exhibits a relative intensity of more than 95% and the peak at 18.4±0.2 (2θ) preferably exhibits a relative intensity of more than 15%.

In another preferred embodiment, the crystalline form according to the present invention is crystalline form H. According to this embodiment, the crystalline form preferably has an X-ray powder diffraction peak (CuK_{α} radiation) at 18.5±0.2 (2θ), more preferably the crystalline form has X-ray powder diffraction peaks (CuK_{α} radiation) at 18.5±0.2 (2θ) and 17.5±0.2 (2θ), still more preferably the crystalline form has X-ray powder diffraction peaks (CuK_{α} radiation) at 18.5±0.2 (2θ), 17.5±0.2 (2θ) and 19.2±0.2 (2θ), most preferably the crystalline form has X-ray powder diffraction peaks (CuK_{α} radiation) at 18.5±0.2 (2θ), 17.5±0.2 (2θ), 19.2±0.2 (2θ) and 9.6±0.2 (2θ), and in particular the crystalline form has X-ray powder diffraction peaks (CuK_{α} radiation) at 18.5±0.2 (2θ), 17.5±0.2 (2θ), 19.2±0.2 (2θ), 9.6±0.2 (2θ) and 15.1±0.2 (2θ). Further according to this embodiment, all aforementioned X-ray powder diffraction peaks preferably exhibit a relative intensity of at least 30%.

Preferably, characteristic X-ray powder diffraction peaks (CuK_{α} radiation) of crystalline form H serving to better differentiate crystalline form H from crystalline forms A, B, C, D, E, F, G, I, J and K, in particular from J, are 17.5±0.2 (2θ) which preferably exhibits a relative intensity of more than 45% and 22.4±0.2 (2θ) which preferably exhibits a relative intensity of more than 25%. Therefore, crystalline form H preferably comprises X-ray powder diffraction peaks (CuK_{α} radiation) at 18.5±0.2 (2θ), 17.5±0.2 (2θ), 19.2±0.2 (2θ) and 22.4±0.2 (2θ), wherein the peak at 17.5±0.2 (2θ) preferably exhibits a relative intensity of more than 45% and the peak at 22.4±0.2 (2θ) preferably exhibits a relative intensity of more than 25%.

In still another preferred embodiment, the crystalline form according to the present invention is crystalline form I. According to this embodiment, the crystalline form preferably has an X-ray powder diffraction peak (CuK_{α} radiation) at 18.1±0.2 (2θ), more preferably the crystalline form has X-ray powder diffraction peaks (CuK_{α} radiation) at 18.1±0.2 (2θ) and 17.6±0.2 (2θ) and most preferably the crystalline form has X-ray powder diffraction peaks (CuK_{α} radiation) at 18.1±0.2 (2θ), 17.6±0.2 (2θ) and 19.1±0.2 (2θ). Further according to this embodiment, all aforementioned X-ray powder diffraction peaks preferably exhibit a relative intensity of at least 45%.

Preferably, characteristic X-ray powder diffraction peaks (CuK_{α} radiation) of crystalline form I serving to better differentiate crystalline form I from crystalline forms A, B, C, D, E, F, G, H, J and K, in particular from C, are 11.8±0.2 (2θ) which preferably exhibits a relative intensity of more than 6% and 27.2±0.2 (2θ) which preferably exhibits a relative intensity of more than 7%. Therefore, crystalline form I preferably comprises X-ray powder diffraction peaks (CuK_{α} radiation) at 18.1±0.2 (2θ), 17.6±0.2 (2θ), 19.1±0.2 (2θ), 11.8±0.2 (2θ) and 27.2±0.2 (2θ), wherein the peak at 11.8±0.2 (2θ) preferably exhibits a relative intensity of more than 6% and the peak at 27.2±0.2 (2θ) preferably exhibits a relative intensity of more than 7%.

In yet another preferred embodiment, the crystalline form according to the present invention is crystalline form J. According to this embodiment, the crystalline form preferably has an X-ray powder diffraction peak (CuK_{α} radiation) at 18.5±0.2 (2θ), more preferably the crystalline form has X-ray powder diffraction peaks (CuK_{α} radiation) at 18.5±0.2 (2θ) and 19.2±0.2 (2θ), most preferably the crystalline form has X-ray powder diffraction peaks (CuK_{α} radiation) at 18.5±0.2 (2θ), 19.2±0.2 (2θ) and 17.5±0.2 (2θ), and in particular the crystalline form has X-ray powder diffraction peaks (CuK_{α} radiation) at 18.5±0.2 (2θ), 19.2±0.2 (2θ), 17.5±0.2 (2θ) and 9.6±0.2 (2θ). Further according to this embodiment, all aforementioned X-ray powder diffraction peaks preferably exhibit a relative intensity of at least 30%.

Preferably, characteristic X-ray powder diffraction peaks (CuK_{α} radiation) of crystalline form J serving to better differentiate crystalline form J from crystalline forms A, B, C, D, E, F, G, H, I and K, in particular from H, are 18.0±0.2 (2θ) which preferably exhibits a relative intensity of more than 20% and 7.9±0.2 (2θ) which preferably exhibits a relative intensity of more than 8%. Therefore, crystalline form J preferably comprises X-ray powder diffraction peaks (CuK_{α} radiation) at 18.5±0.2 (2θ), 19.2±0.2 (2θ), 17.5±0.2 (2θ), 9.6±0.2 (2θ), 18.0±0.2 (2θ) and 7.9±0.2 (2θ), wherein the peak at 18.0±0.2 (2θ) preferably exhibits a relative intensity of more than 20% and the peak at 7.9±0.2 (2θ) preferably exhibits a relative intensity of more than 8%.

In a further preferred embodiment, the crystalline form according to the present invention is crystalline form K. According to this embodiment, the crystalline form preferably has an X-ray powder diffraction peak (CuK_{α} radiation) at 19.0±0.2 (2θ), more preferably the crystalline form has X-ray powder diffraction peaks (CuK_{α} radiation) at 19.0±0.2 (2θ) and 19.6±0.2 (2θ), most preferably the crystalline form has X-ray powder diffraction peaks (CuK_{α} radiation) at 19.0±0.2 (2θ), 19.6±0.2 (2θ) and 19.2±0.2 (2θ), and in particular the crystalline form has X-ray powder diffraction peaks (CuK_{α} radiation) at 19.0±0.2 (2θ), 19.6±0.2 (2θ), 19.2±0.2 (2θ) and 5.9±0.2 (2θ). Further according to this embodiment, all aforementioned X-ray powder diffraction peaks preferably exhibit a relative intensity of at least 40%.

Preferably, characteristic X-ray powder diffraction peaks (CuK_{α} radiation) of crystalline form K serving to better differentiate crystalline form K from crystalline forms A, B, C, D, E, F, G, H, I and J, in particular from G, are 19.6±0.2 (2θ) which preferably exhibits a relative intensity of more than 90% and 19.2±0.2 (2θ) which preferably exhibits a relative intensity of more than 80%. Therefore, crystalline form K preferably comprises X-ray powder diffraction peaks (CuK_{α} radiation) at 19.0±0.2 (2θ), 19.6±0.2 (2θ) and 19.2±0.2 (2θ), wherein the peak at 19.6±0.2 (2θ) preferably exhibits a relative intensity of more than 90% and the peak at 19.2±0.2 (2θ) preferably exhibits a relative intensity of more than 80%.

Preferably, the crystalline form according to the present invention exhibits at least one endothermic event in differential scanning calorimetry (DSC) analysis. A person skilled in the art knows how to realize DSC analysis. Preferably, DSC measurements are realized using a Mettler Toledo DSC821 or Mettler Toledo DSC823 (nitrogen flow, temperature range: -50°C to 350°C, heating rate: 10°C/min).

Preferably, the crystalline form according to the present invention exhibits in differential scanning calorimetry analysis
at least one endothermic event with an onset temperature in the range of from 70°C to 90°C and/or 80°C to 100°C and/or 90°C to 110°C and/or 180°C to 215°C and/or 240°C to 270°C; and/or
a peak temperature in the range of from 80°C to 100°C and/or 90°C to 110°C and/or 105°C to 130°C and/or 190°C to 220°C and/or 245°C to 275°C.

In a particularly preferred embodiment, the crystalline form according to the present invention is crystalline form F exhibiting in differential scanning calorimetry analysis a first endothermic event with an onset temperature in the range of from 180°C to 215°C, more preferably 185°C to 210°C, most preferably 190°C to 208°C and/or a peak temperature in the range of from 190°C to 220°C, more preferably 193°C to 215°C, most preferably 195°C to 213°C; and a second endothermic event with an onset temperature in the range of from 240°C to 270°C, more preferably 245°C to 265°C, most preferably 250°C to 260°C and/or a peak temperature in the range of from 245°C to 275°C, more preferably 250°C to 270°C, most preferably 252°C to 265°C.

In another preferred embodiment, the crystalline form according to the present invention is crystalline form B exhibiting in differential scanning calorimetry analysis a first endothermic event with an onset temperature in the range of from 175°C to 195°C, more preferably 178°C to 192°C, most preferably 181°C to 191°C and/or a peak temperature in the range of from 180°C to 200°C, more preferably 185°C to 199°C, most preferably 187.5°C to 197.5°C; a second endothermic event with an onset temperature in the range of from 235°C to 255°C, more preferably 238°C to 253°C, most preferably 241°C to 251°C and/or a peak temperature in the range of from 240°C to 260°C, more preferably 243°C to 258°C, most preferably 246°C to 256°C; and a third endothermic event with an onset temperature in the range of from 245°C to 265°C, more preferably 247°C to 263°C, most preferably 250°C to 260°C and/or a peak temperature in the range of from 247°C to 267°C, more preferably 162°C to 265°C, most preferably 252°C to 262°C.

In still another preferred embodiment, the crystalline form according to the present invention is crystalline form A exhibiting in differential scanning calorimetry analysis a first endothermic event with an onset temperature in the range of from 70°C to 90°C, more preferably 77°C to 87°C and/or a peak temperature in the range of from 80°C to 110°C, more preferably 87°C to 105°C; a second endothermic event with an onset temperature in the range of from 80°C to 125°C, more preferably 95°C to 123°C and/or a peak temperature in the range of from 105°C to 135°C, more preferably 113°C to 132°C; and a third endothermic event with an onset temperature in the range of from 245°C to 265°C, more preferably 250°C to 263°C, most preferably 252°C to 262°C and/or a peak temperature in the range of from 250°C to 270°C, more preferably 253°C to 267°C, most preferably 255°C to 265°C.

In even another preferred embodiment, the crystalline form according to the present invention is crystalline form C exhibiting in differential scanning calorimetry analysis a first endothermic event with an onset temperature in the range of from 177°C to 200°C, more preferably 180°C to 198°C, most preferably 182°C to 196°C and/or a peak temperature in the range of from 180°C to 217°C, more preferably 183°C to 214°C, most preferably 186°C to 212°C; a second endothermic event with an onset temperature in the range of from 243°C to 263°C, more preferably 246°C to 261°C, most preferably 249°C to 259°C and/or a peak temperature in the range of from 245°C to 265°C, more preferably 247°C to 263°C, most preferably 249°C to 260°C.

In yet another preferred embodiment, the crystalline form according to the present invention is crystalline form D exhibiting in differential scanning calorimetry analysis a first endothermic event with an onset temperature in the range of from 65°C to 90°C, more preferably 68°C to 87°C, most preferably 70°C to 84°C and/or a peak temperature in the range of from 77°C to 104°C, more preferably 80°C to 101°C, most preferably 82°C to 99°C; a second endothermic event with an onset temperature in the range of from 180°C to 200°C more preferably 185°C to 199°C, most preferably 188°C to 198°C or 235°C to 255°C more preferably 238°C to 252°C, most preferably 240°C to 250°C and/or a peak temperature in the range of from 190°C to 210°C more preferably 194°C to 209°C, most preferably 197°C to 207°C or 248°C to 268°C more preferably 250°C to 265°C, most preferably 253°C to 263°C; and a third endothermic event with an onset temperature in the range of from 245°C to 265°C more preferably 248°C to 264°C, most preferably 251°C to 263°C and/or a peak temperature in the range of from 247°C to 267°C more preferably 250°C to 266°C, most preferably 253°C to 265°C.

In a further preferred embodiment, the crystalline form according to the present invention is crystalline form E exhibiting in differential scanning calorimetry analysis a first endothermic event with an onset temperature in the range of from 77°C to 99°C more preferably 80°C to 96°C, most preferably 82°C to 94°C and/or a peak temperature in the range of from 86°C to 110°C more preferably 89°C to 107°C, most preferably 91°C to 104°C; and a second endothermic event with an onset temperature in the range of from 243°C to 265°C more preferably 246°C to 263°C, most preferably 248°C to 260°C and/or a peak temperature in the range of from 245°C to 267°C more preferably 248°C to 265°C, most preferably 249°C to 262°C.

In still a further preferred embodiment, the crystalline form according to the present invention is crystalline form G exhibiting in differential scanning calorimetry analysis a first endothermic event with an onset temperature in the range of from 70°C to 90°C more preferably 73°C to 88°C, most preferably 76°C to 86°C or 80°C to 100°C more preferably 83°C to 98°C, most preferably 86°C to 96°C and/or a peak temperature in the range of from 84°C to 104°C more preferably 87°C to 101°C, most preferably 89°C to 99°C or 95°C to 115°C more preferably 98°C to 112°C, most preferably 100°C to 110°C; an optional exothermic event with an onset temperature in the range of from 200°C to 220°C more preferably 203°C to 218°C, most preferably 206°C to 216°C and/or a peak temperature in the range of from 208°C to 230°C more preferably 210°C to 227°C, most preferably 213°C to 224°C; and a second endothermic event with an onset temperature in the range of from 244°C to 265°C more preferably 247°C to 262°C, most preferably 250°C to 260°C and/or a peak temperature in the range of from 245°C to 266°C more preferably 248°C to 263°C, most preferably 251°C to 261°C.

In yet a further preferred embodiment, the crystalline form according to the present invention is crystalline form H exhibiting in differential scanning calorimetry analysis an endothermic event with an onset temperature in the range of from 244°C to 264°C more preferably 247°C to 261°C, most preferably 249°C to 259°C and/or a peak temperature in the range of from 247°C to 267°C more preferably 249°C to 264°C, most preferably 252°C to 262°C.

In another preferred embodiment, the crystalline form according to the present invention is crystalline form I exhibiting in differential scanning calorimetry analysis an exothermic event with an onset temperature in the range of from 172°C to 192°C more preferably 175°C to 189°C, most preferably 177°C to 187°C and/or a peak temperature in the range of from 184°C to 204°C more preferably 187°C to 201°C, most preferably 189°C to 199°C; and an endothermic event with an onset temperature in the range of from 244°C to 264°C more preferably 247°C to 261°C, most preferably 249°C to 259°C and/or a peak temperature in the range of from 245°C to 265°C more preferably 247°C to 262°C, most preferably 250°C to 260°C.

In still another preferred embodiment, the crystalline form according to the present invention is crystalline form J exhibiting in differential scanning calorimetry analysis an endothermic event with an onset temperature in the range of from 244°C to 264°C more preferably 247°C to 261°C, most preferably 249°C to 259°C and/or a peak temperature in the range of from 245°C to 265°C more preferably 248°C to 262°C, most preferably 250°C to 260°C

It has been surprisingly found that some crystalline forms of the compound according to formula (I) disclosed herein have surprisingly higher stability than other forms, as is demonstrated in the examples. For instance, crystalline form F achieves significantly and surprisingly higher stability, e.g. physical and/or chemical stability than other crystalline forms.

Therefore, in a preferred embodiment, the crystalline form according to the present invention is crystalline form F
having X-ray powder diffraction peaks (CuK_{α} radiation) at 16.9±0.2 (2θ), 16.0±0.2 (2θ) and 24.7±0.2 (2θ); and/or
exhibiting in differential scanning calorimetry analysis a first endothermic event with an onset temperature in the range of from 180°C to 215°C and/or a peak temperature in the range of from 190°C to 220°C; and a second endothermic event with an onset temperature in the range of from 240°C to 270°C and/or a peak temperature in the range of from 245°C to 275°C.

Drug stability plays an important role for pharmaceutical dosage forms. By using the most stable modification of the pharmacologically active ingredient in a pharmaceutical dosage form it may specifically be ensured that, during storage, no crystalline conversion or polymorphic conversion of said pharmacologically active ingredient takes place. This is advantageous, because otherwise the properties of the pharmaceutical dosage form could change as a consequence of a conversion of a less stable modification into a more stable modification. Depending on the pharmacological properties of the pharmaceutical dosage form, this could lead e.g. to changes in the solubility of the pharmacologically active ingredient, accompanied by a change in the release characteristics and thus also a change in the bioavailability. Thus, these effects could result in an inadequately decreased shelf life of the pharmaceutical dosage form.

It has been surprisingly found that crystalline form F which exists as an ansolvate exhibits a high stability which is advantageous for use in pharmaceutical dosage forms. Ansolvate crystalline forms of a pharmacologically active ingredient have advantages due to the fact that they represent the crystalline form having the lowest weight per mol for that pharmacologically active ingredient, thereby reducing the mass of pharmacologically active ingredient required to achieve a certain dosage in a pharmaceutical dosage form, such as a tablet, compared to crystalline forms which bind or form complexes with residual solvent.

Surprisingly, it has also been found that crystalline form F is not hygroscopic.

Further, it has been surprisingly found that crystalline form B which exists as an ansolvate achieves higher stability, e.g. physical and/or chemical stability, than many other crystalline forms.

Therefore, in another preferred embodiment, the crystalline form according to the present invention is crystalline form B
having X-ray powder diffraction peaks (CuK_{α} radiation) at 18.4±0.2 (2θ), 19.1±0.2 (2θ) and 7.9±0.2 (2θ); and/or
exhibiting in differential scanning calorimetry analysis a first endothermic event with an onset temperature in the range of from 175°C to 195°C and/or a peak temperature in the range of from 180°C to 200°C; a second endothermic event with an onset temperature in the range of from 235°C to 255°C and/or a peak temperature in the range of from 240°C to 260°C; and a third endothermic event with an onset temperature in the range of from 245°C to 265°C and/or a peak temperature in the range of from 247°C to 267°C.

Details of the properties of the crystalline forms according to the present invention are disclosed in greater detail in the examples below.

Another aspect of the present invention relates to a process for preparing a crystalline form according to the present invention.

In a preferred embodiment, the process for preparing a crystalline form according to the present invention comprises the steps of
(i) mixing the compound according to formula (I) with a solvent;
(ii) stirring at room temperature or stirring at an elevated temperature the mixture obtained in step (i);
(iii) separating the solid from the solvent; and
(iv) drying the solid obtained in step (iii).

In step (i), suitable solvents are conventional solvents known to persons skilled in the art, such as water or organic solvents selected from the group consisting of alcohols such as methanol, ethanol, n-propanol, 2-propanol and n-butanol; esters such as ethyl acetate, n-propyl acetate, iso-propyl acetate, n-butyl acetate and iso-butyl acetate; ketones such as acetone, methyl ethyl ketone (2-butanone), pentan-2-one, pentan-3-one, hexan-2-one and hexan-3-one; ethers such as methyl *tert*-butyl ether, diethylether, tetrahydrofuran, diisopropylether and 1,4-dioxane; nitriles such as acetonitril; aromatic hydrocarbons such as toluene; saturated hydrocarbons such as n-pentane, n-hexane and n-heptane; chlorinated hydrocarbons such as dichloromethane and chloroform; and also N-methyl-2-pyrrolidone, dimethyl formamide and dimethyl sulfoxide; and mixtures thereof.

In a preferred embodiment, the solvent used in step (i) is selected from the group consisting of ethyl acetate, acetone and acetonitrile.

Step (ii) can be realized at elevated temperatures. In this regard, elevated temperatures shall refer to any temperature above room temperature. In a preferred embodiment, step (ii) is carried out at a temperature below the boiling point of the respective solvent, more preferably at room temperature. In another preferred embodiment, step (ii) is carried out at the boiling point of the respective solvent. In a particularly preferred embodiment, step (ii) is realized at a temperature in the range of from 20°C to 50°C. Preferably, in step (ii), the compound according to formula (I) remains undissolved or is partially dissolved or completely dissolved in the solvent. According to this embodiment, step (ii) gives a suspension or a solution.

In a preferred embodiment, the stirring carried out in step (ii) is realized for a time period of at least 15 min, more preferably in a range of from 15 min to 7 days, still more preferably 15 min to 4 days, even more preferably 15 min to 2 days, yet more preferably 15 min to 1 day, most preferably 15 min to 12 h, and in particular 15 min to 2h.

Optionally, after step (ii) and before step (iii), step (iia) can be carried out. Thus, in a preferred embodiment, the process according to the present invention comprises step (iia) which relates to precipitating the compound according to formula (I). Preferably, step (iia) involves cooling, preferably in an ice bath, of the suspension or solution obtained in step (ii). Cooling is especially preferred, when step (ii) is realized at elevated temperatures and/or when the compound according to formula (I) is completely or partially dissolved in the solvent.

Step (iii) is preferably realized by filtering or by evaporating the solvent, more preferably by filtering.

Step (iv), i.e. the drying of the solid obtained in step (iii), is preferably carried out at ambient conditions. In another preferred embodiment, step (iv) is realized at temperatures in the range of from 25°C to 50°C, preferably in a drying cabinet, and/or under reduced pressure, more preferably at pressures of 0 to 900 mbar, even more preferably 1 to 500 mbar, and in particular 10 to 200 mbar.

Mixtures of the crystalline forms A, B, C, D, E, F, G, H, I, J and K, preferably mixtures of two of these crystalline forms, are also included within the scope of the present invention.

For example, such mixtures of two crystalline forms may be obtained from one or more of the crystalline forms A, B, C, D, E, F, G, H, I, J or K during a crystallization process (e.g. cooling or evaporation) or respectively during a separation process (e.g. filtration), or respectively during a process where heat is applied (e.g. drying), or respectively during a process where mechanical energy is inserted (e.g. milling or grinding).

Furthermore, such mixtures of two crystalline forms may be obtained from one or more of crystalline forms A, B, C, D, E, F, G, H, I, J or K by a partial uptake of hydrate water or respectively by a partial loss of hydrate water, or respectively by a solvent/water exchange.

Another aspect of the present invention relates to a mixture of at least two crystalline forms according to the present invention; or a mixture of at least one crystalline form according to the present invention with an amorphous form; or a mixture of at least one crystalline form according to the present invention with a salt of the compound according to formula (I), in any mixing ratio.

Preferably, the degree of crystallinity, i.e. the amount of crystalline form(s) relative to the total amount of the compound according to formula (I) containing crystalline form(s) and maybe also amorphous form is at least 40 wt.-%, more preferably at least 60 wt.-%, still more preferably at least 80 wt.-%, yet more preferably at least 90 wt.-%, even more preferably at least 95 wt.-%, most preferably at least 99 wt.-%, and in particular at least 99.5 wt.-%.

Another aspect of the present invention relates to a pharmaceutical dosage form comprising at least one crystalline form according to the present invention. In this regard, the term "at least one" shall preferably mean "1, 2, 3, 4, 5, 6, 7, 8, 9, 10 or 11". More preferably, the pharmaceutical dosage form according to the present invention only comprises one crystalline form according to the present invention.

In still another aspect, the present invention relates to methods of treating pain, comprising administering a pharmaceutical dosage form that comprises a crystalline form according to the present invention to a patient in need thereof (for example, a patient who has been diagnosed with a pain disorder).

In a preferred embodiment, the present invention relates to the use of a crystalline form according to the present invention in the treatment of pain, wherein the pain is selected from the group consisting of acute, visceral, neuropathic or chronic pain.

In another aspect the present invention relates to a pharmaceutical dosage form comprising a crystalline form according to the present invention and optionally one or more pharmaceutical excipients.

Preferably, said pharmaceutical dosage form may be used for the treatment of pain.

In a preferred embodiment, the pharmaceutical dosage form is a solid drug form. The pharmaceutical dosage form is preferably manufactured for oral administration. However, other forms of administration are also possible, e.g. for buccal, sublingual, transmucosal and rectal administration.

In a preferred embodiment, the pharmaceutical dosage form comprises a crystalline form according to the present invention and one or more pharmaceutical excipients. Preferred pharmaceutical excipients in the sense of the present invention are all substances known to a person skilled in the art which are suitable for use in galenic formulations. The choice of these pharmaceutical excipients and also their quantities are dependent on how the dosage form is to be administered, i.e. orally, sublingually, buccally, transmucosally or rectally.

Furthermore, the present invention relates to a method for treating pain in a patient, preferably in a mammal, which comprises administering an effective amount of a crystalline form according to the present invention to a patient.

### EXAMPLES

The following examples serve to explain the present invention in more detail, but should not be interpreted as restrictive.

The following abbreviations are used in the examples:
- 1 BuOH: 1-butanol
- c: concentration
- d: day
- DCM: dichloromethane
- EA: ethyl acetate
- Et₂O: diethyl ether
- EtOH: ethanol
- Ex.: example
- h: hour
- MeCN: acetonitril
- MEK: 2-butanone
- MeOH: methanol
- min: minute
- MTBE: methyl *tert*-butyl ether
- 1PrOH: 1-propanol
- 2PrOH: iso-propanol (2-propanol)
- rH: relative humidity
- RT: room temperature, preferably 20-25°C
- rpm: rounds per minute
- sec: seconds
- t: time (duration)
- T: temperature
- XRPD: X-ray powder diffraction

In the following "compound (1)" denotes the compound according to formula (I) which synthesis is described in WO 2004/043967 (see example 49).

XRPD analyses were carried out in transmission geometry with a STOE StadiP or a Panalytical X'Pert Pro X-ray powder diffractometer in reflection geometry, monochromatised CuK_{α} radiation being used by means of a germanium monocrystal. Measurements were carried out in an angular range between 5° and 50° in 2θ. In general, the 2θ values have an error rate of ±0.2° in 2θ. The samples were measured without any special treatment other than the application of slight pressure to get a flat surface. An ambient air atmosphere was used. Unless stated otherwise, measurements were performed at RT (i.e. 298 K (± 5 K)). In general a baseline correction of the measured diffractograms was done using the program WinXPow (STOE).

### A) Crystalline form A

### XRPD

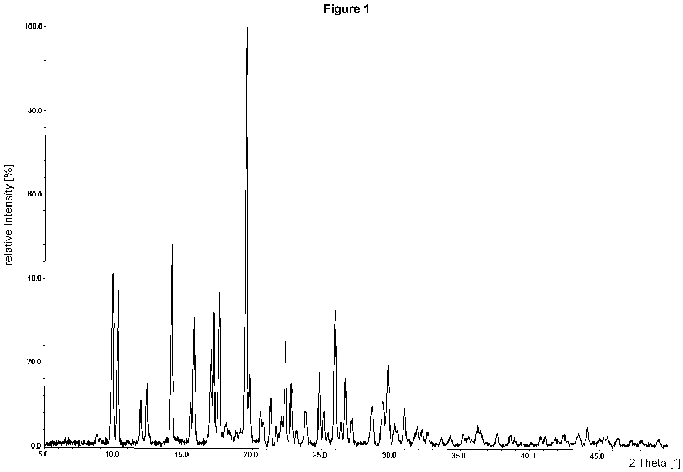

Figure 1 shows the XRPD analysis of crystalline form A.

In Table 1 below, the peak list of crystalline form A is summarized. Maximum relative intensity is 100.

**Table 1:**

| **2θ** | **rel I** | **2θ** | **rel I** |
|---|---|---|---|
| 5.8601 | 43.29 | 19.6144 | 92.85 |
| 9.8601 | 8.9 | 20.04 | 6.71 |
| 11.2817 | 6.92 | 20.4402 | 5.34 |
| 11.4671 | 13.67 | 21.3566 | 8.12 |
| 13.3919 | 5.44 | 22.0271 | 23.68 |
| 14.8764 | 2.88 | 23.0388 | 4.87 |
| 15.3056 | 4.08 | 23.7639 | 18.89 |
| 15.7284 | 3.4 | 20.7519 | 0.44 |
| 16.6172 | 9.95 | 28.0751 | 2.76 |
| 17.2827 | 4.47 | 29.1725 | 14.54 |
| 17.801 | 7.92 | 29.6805 | 10.35 |
| 19.0461 | 100 | 37.7023 | 12.43 |
| 19.239 | 84.5 | | |

### Synthesis of crystalline form A

### Example A1:

A vial was charged with compound (1) (crystalline form A, 50 mg) and MeOH (10 mL). The mixture was vortexed in an Eppendorf Thermomixer for 4 h at 30°C and 400 rpm. Afterwards, the solvent was evaporated at 23°C. The solid was determined by XRPD to be crystalline form A.

### Example A2:

A vial was charged with compound (1) (crystalline form C, 53.4 mg) and MeOH (3 mL). The mixture was vortexed in an Eppendorf Thermomixer for 18 h at 30°C and 700 rpm. Afterwards, the solid was separated by filtration and dried in air. The solid was determined by XRPD to be crystalline form A.

### Example A3:

A vial was charged with compound (1) (crystalline form J, 101 mg) and MeOH (5 mL). The mixture was vortexed in an Eppendorf Thermomixer for 16 h at 30°C and 700 rpm. Afterwards, the mixture was stirred for 1 h at 21°C. The solid was separated and dried using a suction filter (5 sec) and was determined by XRPD to be crystalline form A.

### Example A4:

A vial was charged with compound (1) (crystalline form C, 200 mg) and MeOH (2.5 mL). The mixture was vortexed in an Eppendorf Thermomixer for 72 h at 25°C and 400 rpm. Afterwards, the mixture was filtered using a suction filter and the solid was dried by further suction of air through the filter cake for app. 5 min. The solid was determined by XRPD to be crystalline form A.

### B) Crystalline form B

### XRPD

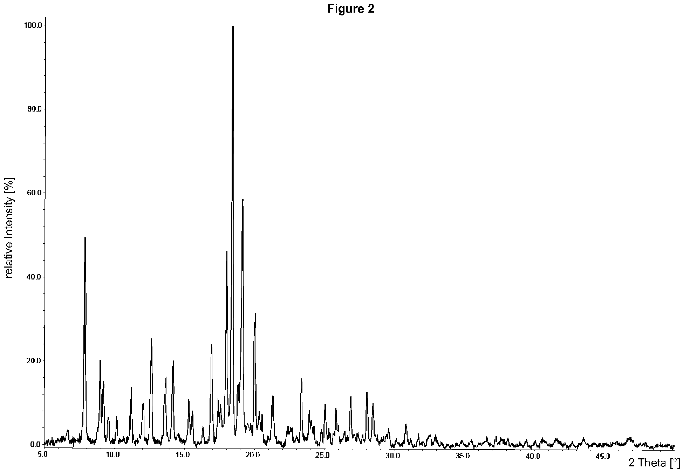

Figure 2 shows the XRPD analysis of crystalline form B.

In Table 2 below, the peak list of crystalline form B is summarized. Maximum relative intensity is 100.

**Table 2:**

| **2θ** | **rel I** | **2θ** | **rel I** | **2θ** | **rel I** |
|---|---|---|---|---|---|
| 6.6556 | 2.61 | 19.1082 | 57.61 | 26.0435 | 1.35 |
| 7.8808 | 50.42 | 19.5294 | 4.04 | 27.1302 | 1.9 |
| 8.8102 | 2.63 | 19.7404 | 2.89 | 27.4019 | 2.2 |
| 8.9898 | 20.1 | 20.0129 | 31.16 | 27.7293 | 1.85 |
| 9.2022 | 14.75 | 20.3319 | 6.13 | 28.0427 | 12.75 |
| 9.5547 | 6.62 | 20.5484 | 6.61 | 28.4658 | 9.24 |
| 10.1622 | 6.28 | 20.9771 | 1.82 | 29.4909 | 2.35 |
| 11.1964 | 13.61 | 21.3096 | 11.44 | 30.8272 | 4.57 |
| 12.0476 | 8.67 | 22.4002 | 3.48 | 31.6928 | 2.36 |
| 12.6254 | 24.66 | 22.6391 | 3.62 | 32.4879 | 2.42 |
| 13.6341 | 15.6 | 23.0108 | 1.74 | 32.9469 | 2.4 |
| 14.1685 | 19.09 | 23.3618 | 14.97 | 36.5922 | 1.36 |
| 15.3326 | 9.48 | 23.9213 | 7.21 | 37.2454 | 1.78 |
| 15.5687 | 7.99 | 24.0771 | 4.86 | 38.0888 | 1.47 |
| 16.3303 | 4.19 | 24.2196 | 3.63 | 39.4108 | 1.27 |
| 16.9344 | 24.09 | 24.7868 | 3.22 | 40.0388 | 1.11 |
| 17.4039 | 8.85 | 25.0586 | 8.93 | 41.5933 | 1.56 |
| 17.5919 | 7.19 | 25.3328 | 2.57 | 42.6823 | 1.06 |
| 17.9854 | 42.81 | 25.8366 | 6.83 | 43.4845 | 1.63 |
| 18.3948 | 100 | 26.437 | 2.02 | 46.7807 | 1.69 |
| 18.8407 | 11.26 | 26.8734 | 10.99 | | |

### Synthesis of crystalline form B

### Example B1:

A vial was charged with compound (1) (crystalline form A, 50 mg) and solvent (10 mL). The mixture was vortexed in an Eppendorf Thermomixer for 4 h at 30°C and 400 rpm. Afterwards, the solvent was evaporated at 23°C. In Table 3 below, used solvents are summarized.

**Table 3:**

| **Ex.** | **solvent** | **Ex.** | **solvent** |
|---|---|---|---|
| B1-1 | DCM | B1-7 | Et₂O |
| B1-2 | n-hexane | B1-8 | MTBE |
| B1-3 | n-pentane | B1-9 | acetone |
| B1-4 | 2PrOH | B1-10 | MEK |
| B1-5 | toluene | B1-11 | THF |
| B1-6 | EA | B1-12 | MeCN |

All of Examples B1-1 to B1-12 gave a solid that was determined by XRPD to be crystalline form B.

### Example B2:

Vials were charged with compound (1) (crystalline form C) and solvent. The mixtures were vortexed using a PLS Synthesizer for 1.5 h at 50°C and 400 rpm. Afterwards, Ex. B2-1 to B2-3 were stored for 120 h in a freezer at -18°C before the respective solvent was evaporated with pressured air at 23°C. In case of Ex. B2-4 to B2-10, the mixtures were left over night at 30°C without shaking before the respective solvent was evaporated with pressured air. In Table 4 below, details are summarized.

**Table 4:**

| **Ex.** | **solvent** | | **compound (1)** |
|---|---|---|---|
| | type | V [mL] | m (weighed in) [mg] |
| B2-1 | EA | 2 | 30.5 |
| B2-2 | MTBE | 3 | 30.1 |
| B2-3 | THF | 0.2 | 30.6 |
| B2-4 | DCM | 1 | 30.9 |
| B2-5 | 1 PrOH | 3 | 30.6 |
| B2-6 | EA | 2 | 31.4 |
| B2-7 | MTBE | 3 | 30.8 |
| B2-8 | acetone | 1 | 31.7 |
| B2-9 | MEK | 0.5 | 31.0 |
| B2-10 | THF | 0.2 | 30.5 |

All of Examples B2-1 to B2-10 gave a solid that was determined by XRPD to be crystalline form B.

### Example B3:

Vials were charged with compound (1) (crystalline form C) and solvent (10 mL). The mixtures were vortexed using a PLS Synthesizer for 3 h at 40°C and 400 rpm. Afterwards, the mixtures were left for 3 d at 23°C without shaking. Then, the solvents were evaporated with pressured air at 40°C. In Table 5 below, details are summarized.

**Table 5:**

| **Ex.** | **solvent** | **compound (1)** |
|---|---|---|
| | type | m (weighed in) [mg] |
| B3-1 | toluene | 31.0 |
| B3-2 | EA | 31.4 |
| B3-3 | MTBE | 30.8 |
| B3-4 | acetone | 30.4 |
| B3-5 | MEK | 31.8 |
| B3-6 | THF | 31.7 |

All of Examples B3-1 to B3-6 gave a solid that was determined by XRPD to be crystalline form B.

### Example 84:

Vials were charged with compound (1) (crystalline form C) and MeCN. In Table 6 below, details are summarized.

**Table 6:**

| **Ex.** | **MeCN** | **compound (1)** | **conditions** | |
|---|---|---|---|---|
| | V [mL] | m (weighed in) [mg] | reaction | work-up |
| B4-1 | 35 | 35 | 23°C, ultra sonic, 20 sec | evaporation of MeCN at 22°C in fume hood |
| B4-2 | 30 | 100 | 40°C, 400 rpm, 6 d | |

The solids obtained from Ex. B4-1 and Ex. B4-2 were harvested after 4.5 weeks and 4 weeks, respectively. Both Ex. B4-1 and B4-2 gave a solid that was determined by XRPD to be crystalline form B.

### Example B5:

For Ex. B5-1 to B5-3, a solution was prepared from compound (1) (crystalline form C, 200.08 mg) and THF (1 mL). Of this solution, 250 µL (amounts to 50 mg of compound (1)) were charged into vials.

For Ex. B5-4, a solution was prepared from compound (1) (crystalline form C, 149.91 mg) and DCM (10 mL). Of this solution, 3.33 mL (amounts to 50 mg of compound (1)) were charged into a vial.

To all examples, antisolvent was added until precipitation/turbidity was observed. Afterwards, the mixtures were filtered using a G4 suction filter. In Table 7 below, details are summarized.

**Table 7:**

| **Ex.** | **antisolvent** | | **observations** |
|---|---|---|---|
| | type | V [mL] | |
| B5-1 | hexane | 2.0 | turbid after 1.25 mL, solid formed after 2 mL |
| B5-2 | pentane | 1.5 | turbid after 0.8 mL, solid formed after 1.5 mL |
| B5-3 | water | 0.3 | turbid, crystals formed after 15 min |
| B5-4 | pentane | 4.0 | clear solution, crystallization started after 15 min |

All of Examples B5-1 to B5-4 gave a solid that was determined by XRPD to be crystalline form B.

### Example B6:

Compound (1) was ground in a ball mill. In Table 8 below, details are summarized.

**Table 8:**

| **Ex.** | **compound (1)** | | **conditions** |
|---|---|---|---|
| | m (weighed in) [mg] | crystalline form (starting point) | |
| B6-1 | 54.6 | C | 60 min, 20 Hz |
| B6-2 | 500.6 | B+C | 20 min, 20 Hz |
| B6-3 | 500 | B+C | 12 h, 30 Hz |

All of Examples B6-1 to B6-3 gave a solid that was determined by XRPD to be crystalline form B.

### Example B7:

Compound (1) (mixture of crystalline forms B and C, 498 mg) was charged into a Schlenk tube equipped with a cold finger. After evacuation (1 to 2 mbar), the tube was put into an oil bath that was heated to 130°C. After reaching 130°C, the temperature was maintained for 1 h. Then, the temperature was raised to 170°C (held for app. 1 h) and 185°C (held for 2 h). The solid was recovered and determined by XRPD to be crystalline form B.

### Example B8:

Compound (1) (crystalline form B) was stored for 48 h at -40°C. Afterwards, it was determined by XRPD that the crystalline form had been retained (crystalline form B).

### Example B9:

Compound (1) (crystalline form B) was stored for 48 h in a drying cabinet at 23°C and 5 to 7 mbar. Afterwards, it was determined by XRPD that the crystalline form had been retained (crystalline form B).

### Example B10:

Vials were charged with compound (1) (crystalline form H) and solvent. The mixtures were vortexed using a PLS Synthesizer for 16 h at 30°C and 400 rpm. After cooling to RT, the solids were filtered off and the mother liquors were evaporated with pressured air. In Table 9 below, details are summarized.

**Table 9:**

| **Ex**. | **solvent** | | **compound (1)** | |
|---|---|---|---|---|
| | type | V [mL] | m (weighed in) [mg] | yield [wt.-%] |
| B10-1 | acetone | 20 | 109.2 | 84 |
| B10-2 | EA | 20 | 110.3 | 76 |

Both Examples B10-1 and B10-2 gave a solid that was determined by XRPD to be crystalline form B.

### C) Crystalline form C

### XRPD

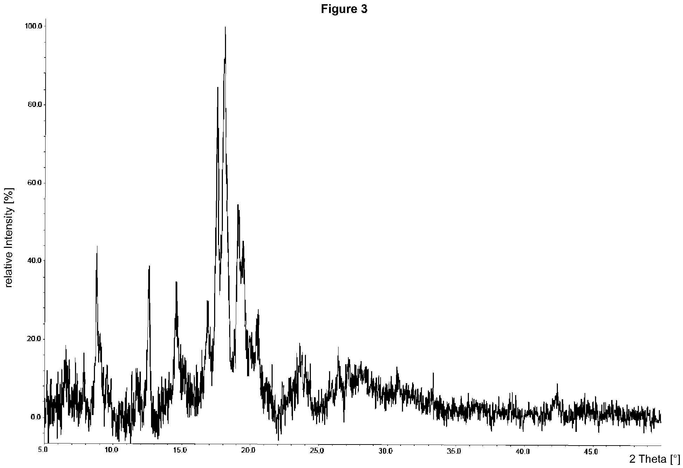

Figure 3 shows the XRPD analysis of crystalline form C.

In Table 10 below, the peak list of crystalline form C is summarized. Maximum relative intensity is 100.

**Table 10:**

| **2θ** | **rel I** | **2θ** | **rel I** |
|---|---|---|---|
| 6.6378 | 4.01 | 17.5564 | 79.94 |
| 7.8176 | 4.95 | 18.0858 | 100 |
| 7.866 | 12.92 | 19.1045 | 47.61 |
| 8.7853 | 42.84 | 19.4563 | 38.66 |
| 9.0587 | 19.21 | 20.0178 | 11.67 |
| 9.5467 | 12.44 | 20.523 | 21.56 |
| 12.5856 | 40.14 | 23.6786 | 13.48 |
| 14.6257 | 26.9 | 26.4468 | 9.77 |
| 16.8741 | 21.66 | 42.3253 | 5.43 |

### Synthesis of crystalline form C

### Example C1:

A vial was charged with compound (1) (crystalline form I, 101 mg) and water (2.25 mL). The mixture was vortexed in an Eppendorf Thermomixer for 72 h at 25°C and 400 rpm. Afterwards, the solid was separated and dried using a suction filter (10 min) and was determined by XRPD to be crystalline form C.

### Example C2:

A vial was charged with compound (1) (crystalline form C, 29.9 mg) and hexane (20 mL). The mixture was vortexed in an Eppendorf Thermomixer for 1.5 h at 50°C and 400 rpm. Afterwards, the solid was stored in the freezer at -18°C for 120 h. Then, when the mixture had gained RT, the solid was separated by filtration and dried in air. The solid was determined by XRPD to be crystalline form C.

### Example C3:

Compound (1) (amorphous) was stored for 48 h at -40°C. Afterwards, it was determined by XRPD that the solid was now crystalline having crystalline form C.

### Example C4:

A vial was charged with compound (1) (crystalline form C, 200 mg) and water (2.5 mL). The mixture was vortexed in an Eppendorf Thermomixer for 72 h at 25°C and 400 rpm. Afterwards, the mixture was filtered using a suction filter and the solid was dried by further suction of air through the filter cake for app. 5 min. The solid was determined by XRPD to be crystalline form C.

### D) Crystalline form D

### XRPD

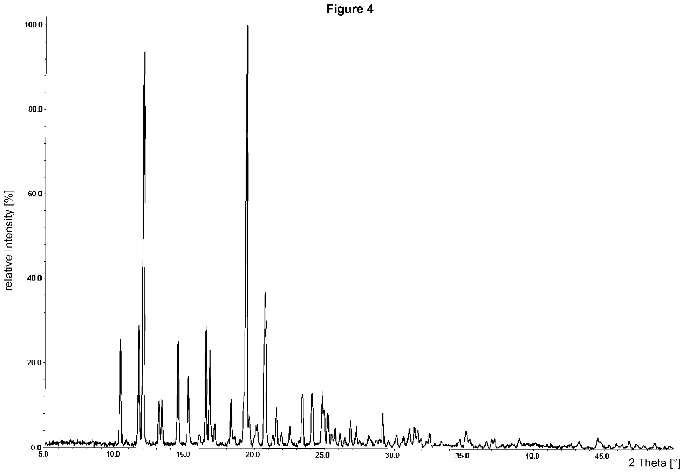

Figure 4 shows the XRPD analysis of crystalline form D.

In Table 11 below, the peak list of crystalline form D is summarized. Maximum relative intensity is 100.

**Table 11:**

| **2θ** | **rel I** | **2θ** | **rel I** | **2θ** | **rel I** |
|---|---|---|---|---|---|
| 10.3926 | 25.29 | 20.7455 | 37.64 | 30.156 | 2.56 |
| 10.8386 | 0.9 | 21.3152 | 2.53 | 30.665 | 1.61 |
| 11.7122 | 27.76 | 21.5712 | 9.33 | 31.0923 | 3.26 |
| 12.0388 | 92.83 | 21.9318 | 2.68 | 31.4643 | 4.04 |
| 13.1626 | 10.23 | 22.5475 | 4.14 | 31.6791 | 3.29 |
| 13.3828 | 10.91 | 23.4352 | 12.73 | 31.9029 | 1.42 |
| 14.5124 | 24.3 | 24.1344 | 12.15 | 32.333 | 0.81 |
| 15.2554 | 15.31 | 24.8505 | 12.04 | 32.5345 | 2.8 |
| 16.0522 | 2.25 | 24.9972 | 8 | 33.3923 | 0.9 |
| 16.5164 | 27.91 | 25.2637 | 7.74 | 34.673 | 1.31 |
| 16.8001 | 20.97 | 25.5334 | 2.58 | 35.1948 | 2.73 |
| 17.1628 | 4.91 | 25.7554 | 3.71 | 36.6154 | 1.29 |
| 18.3269 | 9.35 | 26.1298 | 2.89 | 37.0017 | 1.73 |
| 18.5837 | 1.27 | 26.4592 | 1.45 | 37.198 | 1.69 |
| 18.3695 | 1.56 | 26.8734 | 5.86 | 38.9731 | 1.74 |
| 18.9866 | 1.07 | 27.2851 | 4.22 | 43.2429 | 1.2 |
| 19.2373 | 10.14 | 28.2222 | 1.75 | 44.6534 | 1.68 |
| 19.3938 | 100 | 28.9098 | 0.87 | 45.946 | 0.81 |
| 19.6302 | 6.37 | 29.189 | 7.62 | 46.8266 | 1.16 |
| 20.1424 | 4.68 | 29.6117 | 0.99 | 48.6739 | 1.15 |

### Synthesis of crystalline form D

### Example D1:

A vial was charged with compound (1) (crystalline form A, 50 mg) and 2PrOH (10 mL). The mixture was vortexed in an Eppendorf Thermomixer for 4 h at 30°C and 400 rpm. Afterwards, the solvent was evaporated at 23°C. The solid was determined by XRPD to be crystalline form D.

### E) Crystalline form E

### XRPD

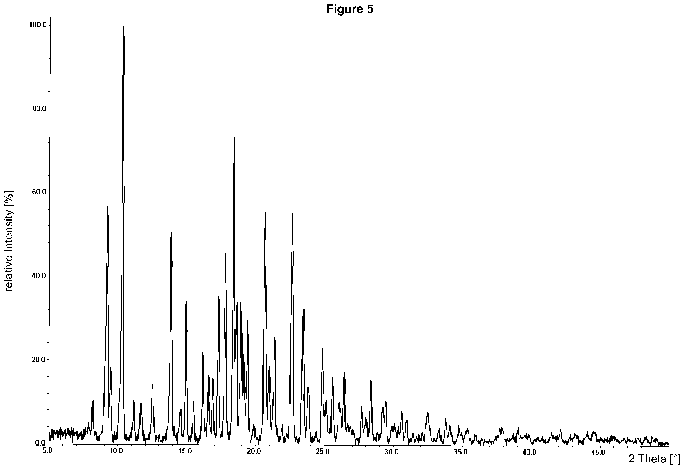

Figure 5 shows the XRPD analysis of crystalline form E.

In Table 12 below, the peak list of crystalline form E is summarized. Maximum relative intensity is 100.

**Table 12:**

| **2θ** | **rel I** | **2θ** | **rel I** | **2θ** | **rel I** |
|---|---|---|---|---|---|
| 8.1736 | 8.62 | 19.8771 | 3.16 | 30.3883 | 2.65 |
| 9.1874 | 53.34 | 20.6483 | 55.29 | 30.612 | 6.96 |
| 9.4768 | 17.05 | 20.9787 | 16.87 | 30.9576 | 4.76 |
| 10.3137 | 100 | 21.3736 | 23.19 | 32.5031 | 5.92 |
| 11.1614 | 8.49 | 21.9194 | 3.24 | 33.2922 | 2.36 |
| 11.6896 | 8.45 | 22.6162 | 55.87 | 33.8187 | 4.42 |
| 12.5186 | 13.05 | 23.4316 | 33.08 | 33.433 | 0.39 |
| 13.8376 | 48.31 | 23.829 | 14.19 | 34.781 | 3.13 |
| 14.5347 | 6.8 | 24.8514 | 22.15 | 35.3556 | 2.61 |
| 14.9536 | 33 | 25.1217 | 8.92 | 37.8115 | 2.83 |
| 15.4994 | 8.81 | 25.5893 | 15.16 | 39.2885 | 1.63 |
| 16.1708 | 19.96 | 26.0442 | 8.28 | 40.8329 | 1.37 |
| 16.5934 | 14.89 | 26.1847 | 6.48 | 41.5828 | 1.41 |
| 16.8961 | 14.57 | 26.4198 | 16.6 | 42.1511 | 2.73 |
| 17.3053 | 36.8 | 26.8075 | 3 | 43.2151 | 1.62 |
| 17.7818 | 45.3 | 27.6996 | 7.34 | 44.0847 | 2.01 |
| 18.3809 | 69.73 | 28.0024 | 4.73 | 44.5768 | 2.45 |
| 18.6011 | 31.82 | 28.3785 | 14.63 | 48.3206 | 1.31 |
| 18.9268 | 35.44 | 29.2382 | 7.98 | 48.8039 | 1.14 |
| 19.1435 | 20.89 | 29.4548 | 8.55 | | |
| 19.4088 | 29.57 | 30.0422 | 3.47 | | |

### Synthesis of crystalline form E

### Example E1:

A vial was charged with compound (1) (crystalline form C, 33.1 mg) and EtOH (5 mL). The mixture was vortexed in an Eppendorf Thermomixer for 1.5 h at 50°C and 400 rpm. Afterwards, the solid was stored in the freezer at -18°C for 120 h. Then, when the mixture had gained RT, the solid was separated by filtration and dried in air. The solid was determined by XRPD to be crystalline form E.

### Example E2:

Vials were charged with compound (1) (crystalline form C, 31.6 mg) and EtOH (4 mL). The mixtures were vortexed using a PLS Synthesizer for 1.5 h at 50°C and 400 rpm. Afterwards, the mixtures were left over night at 30°C without shaking before the respective solvent was evaporated with pressured air. The solid was determined by XRPD to be crystalline form E.

### Example E3:

A vial was charged with compound (1) (crystalline form J, 101 mg) and EtOH (5 mL). The mixture was vortexed in an Eppendorf Thermomixer for 16 h at 30°C and 700 rpm. Afterwards, the mixture was stirred for 1 h at 21°C. The solid was separated and dried using a suction filter (5 sec) and was determined by XRPD to be crystalline form E.

### Example E4:

A vial was charged with compound (1) (crystalline form C, 104.3 mg) and EtOH (2 mL). The mixture was vortexed in an Eppendorf Thermomixer for 68 h at 40°C and 700 rpm. Afterwards, the solid was separated using a suction filter, dried in air and was determined by XRPD to be crystalline form E.

### Example E5:

A vial was charged with compound (1) (crystalline form C, 200 mg) and EtOH (2.5 mL). The mixture was vortexed in an Eppendorf Thermomixer for 72 h at 25°C and 400 rpm. Afterwards, the mixture was filtered using a suction filter and the solid was dried by further suction of air through the filter cake for app. 5 min. The solid was determined by XRPD to be crystalline form E.

### F) Crystalline form F

### XRPD

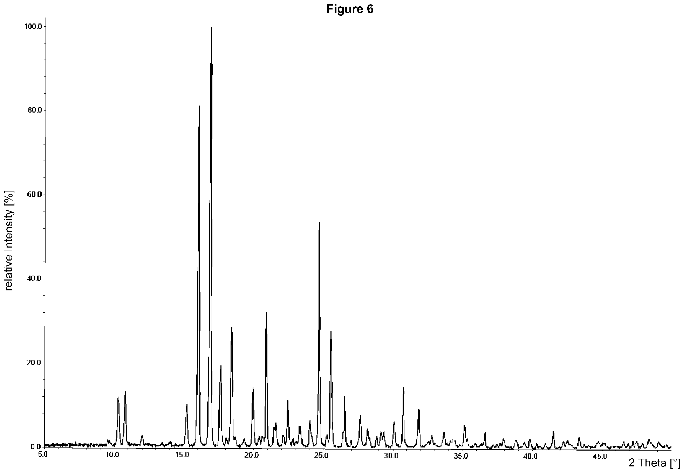

Figure 6 shows the XRPD analysis of crystalline form F.

In Table 13 below, the peak list of crystalline form F is summarized. Maximum relative intensity is 100.

**Table 13:**

| **2θ** | **rel I** | **2θ** | **rel I** | **2θ** | **rel I** |
|---|---|---|---|---|---|
| 10.3104 | 11.57 | 23.3417 | 4.89 | 36.6055 | 3.07 |
| 10.7899 | 12.64 | 24.0776 | 5.53 | 37.2004 | 0.72 |
| 12.0026 | 2.41 | 24.7009 | 46.35 | 37.9469 | 1.88 |
| 13.428 | 0.64 | 25.2711 | 2.6 | 38.8408 | 1.9 |
| 14.0035 | 0.76 | 25.5644 | 28.42 | 39.4365 | 1.23 |
| 15.2068 | 10.07 | 26.5421 | 9.53 | 39.847 | 2.34 |
| 16.0275 | 81.37 | 27.0006 | 1.45 | 40.3254 | 1.04 |
| 16.8781 | 100 | 27.6655 | 7.35 | 40.9653 | 0.98 |
| 17.626 | 19.43 | 28.2252 | 3.62 | 41.5146 | 3.41 |
| 18.0635 | 1.75 | 28.8369 | 2.3 | 42.2353 | 1.45 |
| 18.4244 | 28.87 | 29.1608 | 3.61 | 42.5769 | 1.27 |
| 18.6904 | 1.84 | 29.3566 | 3.15 | 43.3724 | 2.36 |
| 19.3249 | 1.58 | 30.0933 | 5.9 | 44.8831 | 0.9 |
| 19.9721 | 13.45 | 30.7528 | 13.34 | 46.5645 | 1.23 |
| 20.4017 | 2.13 | 31.8576 | 8.2 | 46.8874 | 0.65 |
| 20.6486 | 2.09 | 32.5598 | 0.89 | 47.2349 | 1.51 |
| 20.9177 | 32.22 | 32.8054 | 2.23 | 47.4854 | 1.37 |
| 21.5598 | 4.99 | 33.6765 | 3.31 | 47.9026 | 0.82 |
| 22.1488 | 2.67 | 35.1585 | 4.92 | 48.4217 | 1.74 |
| 22.4705 | 11.09 | 35.922 | 0.78 | 49.0878 | 1.18 |
| 22.8384 | 1.66 | 36.3368 | 0.74 | | |

### Synthesis of crystalline form F

### Example F1:

Vials were charged with compound (1) (crystalline form C) and solvent (10 mL). The mixtures were vortexed using a PLS Synthesizer for 3 h at 40°C and 400 rpm. Afterwards, the mixtures were left for 3 d at 23°C without shaking. Then, the solvents were evaporated with pressured air at 40°C. In Table 14 below, details are summarized.

**Table 14:**

| **Ex.** | **solvent** | **compound (1)** |
|---|---|---|
| | type | m (weighed in) [mg] |
| F1-1 | hexane | 31.2 |
| F1-2 | pentane | 30.9 |

Both examples gave a solid that was determined by XRPD to be crystalline form F.

### Example F2:

Vials were charged with compound (1) (crystalline form H) and solvent. In Table 15 below, details are summarized.

**Table 15:**

| **Ex.** | **solvent** | **compound (1)** | **conditions** | |
|---|---|---|---|---|
| | | | reaction | work-up |
| F2-1 | Et₂O (20 mL) | 128.2 mg (weighed in) 41.3 wt.-% (yield) | 35°C, 400 rpm, 16 h; 23°C, 0 rpm, 1 h | filtration (G4 suction filter), drying by suction of air through filter for 60 min |
| F2-2 | THF (3 mL) MeCN (15 mL) | 112.2 mg (weighed in) 72.6 wt.-% (yield) | RT, 400 rpm, ca. 16h; concentration of solution with pressurized air | |

Both Ex. F1-1 and F2-2 gave a solid that was determined by XRPD to be crystalline form F.

### Example F3:

A vial was charged with compound (1) (crystalline form I, 100.05 mg) and Et₂O (13 mL). The mixture was vortexed using a PLS Synthesizer for 4 h at 35°C and 400 rpm. Afterwards, the mixture was left for 1 h at 23°C without shaking. Then, the solid was filtered (G4 suction filter) and dried by suction of air through the filter cake for 5 min (yield: 38.8 wt.-%). The solid was determined by XRPD to be crystalline form F.

### Example F4:

For Ex. F4-1 a solution was prepared from compound (1) (crystalline form C, 200.08 mg) and THF (1 mL). Of this solution, 250 µL (amounts to 50 mg of compound (1)) was charged into a vial.

For Ex. F4-2, a solution was prepared from compound (1) (crystalline form C, 149.91 mg) and DCM (10 mL). Of this solution, 3.33 mL (amounts to 50 mg of compound (1)) were charged into a vial.

For Ex. F4-3, a vial was charged with compound (1) (crystalline form I, 101.48 mg) and THF (1 mL).

To all examples, antisolvent was added until precipitation/turbidity was observed. Afterwards, the mixtures were filtered using a G4 suction filter. In Table 16 below, details are summarized.

**Table 16:**

| **Ex.** | **antisolvent** | | **observations** |
|---|---|---|---|
| | type | V [mL] | |
| F4-1 | MeCN | 3.5 | solution, after 15 min at RT a solid formed |
| F4-2 | hexane | 4.0 | solution, after 15 min at RT a solid formed; mixture was left at RT for 16 h |
| F4-3 | MeCN | 8 | solution, after 5 min a solid formed; then 2 h at 25°C and 500 rpm |

All of Examples F4-1 to F4-3 gave a solid that was determined by XRPD to be crystalline form F.

*Example F5:*

Vials were charged with compound (1) (crystalline form C) and solvent (3 mL). The mixtures were vortexed in an Eppendorf Thermomixer for 18 h at 30°C and 700 rpm. Afterwards, the solids were separated by filtration and dried in air. Details are summarized in the table below.

**Table 17:**

| **Ex.** | **solvent** | **compound (1)** |
|---|---|---|
| | type | m (weighed in) [mg] |
| F5-1 | MeCN | 50.3 |
| F5-2 | hexane | 51.4 |
| F5-3 | pentane | 53.7 |

All examples gave a solid that was determined by XRPD to be crystalline form F.

### Example F6:

Vials were charged with compound (1) (crystalline form I) and solvent (1.5 mL). The mixtures were vortexed in an Eppendorf Thermomixer for 20 h at 40°C and 1000 rpm and then for 1 h at 23 °C and 600 rpm. Afterwards, the solids were separated by filtration (G4 suction filter) and dried in air. Details are summarized in the table below.

**Table 18:**

| **Ex.** | **solvent** | **compound (1)** | |
|---|---|---|---|
| | type | m (weighed in) [mg] | yield [wt.-%] |
| F6-1 | MeCN | 101.1 | 91.0 |
| F6-2 | MeOH | 101.2 | 40.1 |

All examples gave a solid that was determined by XRPD to be crystalline form F.

### Example F7:

A vial was charged with compound (1) (crystalline form F, 1.02336 g) and acetone (3 mL). The mixture was vortexed in an Eppendorf Thermomixer for 20 h at 23°C and 750 rpm and was then left for 30 h at 23 °C without vortexing. Afterwards, the solids were separated by filtration and dried in air. The solid was determined by XRPD to be crystalline form F.

### Example F8:

Vials were charged with compound (1) and solvent (1 mL).

For F8-1 to F8-13: The mixtures were vortexed in an Eppendorf Thermomixer for 120 h at 40°C and 1000 rpm, then cooled down to 20°C and vortexed for 2 h at 20 °C and 1000 rpm. For F8-14 to F8-20: The mixtures were vortexed in an Eppendorf Thermomixer for 12 d at 40°C and 1000 rpm, then cooled down to RT and kept for 24 h without vortexing at RT.

For F8-21 to F8-29: The mixtures were vortexed in an Eppendorf Thermomixer for 113 h at 40°C and 1000 rpm, then cooled down to 20°C and vortexed for 2 h at 20 °C and 1000 rpm.

For F8-30 to F8-33: Different charges of crystalline form F were used as starting material. The mixtures were vortexed in an Eppendorf Thermomixer for 72 h at 23° (F8-30 and F8-31) or 40°C (F8-32 and F8-33) and 1000 rpm, then cooled down to 20°C and vortexed for 1 h at 20 °C and 1000 rpm.

Then, the solids were separated by filtration and dried in air. Details are summarized in the table below.

**Table 19:**

| **Ex.** | **compound (1)** | | | **solvent** |
|---|---|---|---|---|
| | crystalline form (m [mg]) | crystalline form (m [mg]) | Cₜₒₜₐₗ [mg/mL] | |
| F8-1 | H (27.7) | B (30.0) | 57.7 | acetone |
| F8-2 | F (17.7) | B (17.5) | 35.2 | acetone |
| F8-3 | C (19.8) | B (22.0) | 41.8 | acetone |
| F8-4 | I (22.6) | B (23.1) | 45.7 | acetone |
| F8-5 | H (22.2) | C (20.4) | 42.6 | acetone |
| F8-6 | H (23.0) | I (21.4) | 44.4 | acetone |
| F8-7 | I (27.0) | H (26.9) | 53.8 | EA |
| F8-8 | I (26.2) | C (28.4) | 54.5 | EA |
| F8-9 | I (25.3) | B (25.0) | 50.3 | EA |
| F8-10 | I (27.2) | J (27.2) | 54.4 | EA |
| F8-11 | H (26.0) | C (26.6) | 51.6 | EA |
| F8-12 | E (51.9) | - | 51.9 | EA |
| F8-13 | A (60.5) | - | 60.5 | EA |
| F8-14 | I (27.0) | H (26.9) | 53.8 | MeCN |
| F8-15 | I (26.2) | C (28.4) | 54.5 | MeCN |
| F8-16 | I (25.3) | B (25.0) | 50.3 | MeCN |
| F8-17 | I (27.2) | B (27.2) | 54.4 | MeCN |
| F8-18 | I+B (30.4) | C (31.9) | 62.3 | MeCN |
| F8-19 | G (31.7) | C (30.1) | 61.8 | MeCN |
| F8-20 | G (32.7) | H (33.0) | 65.8 | MeCN |
| F8-21 | B (24.8) | H (24.3) | 49.1 | MeCN |
| F8-22 | C (25.6) | F (25.5) | 51.1 | MeCN |
| F8-23 | C (24.5) | H (24.3) | 48.8 | MeCN |
| F8-24 | C (24.7) | I (25.0) | 49.7 | MeCN |
| F8-25 | C (25.0) | J (23.4) | 48.4 | MeCN |
| F8-26 | F (24.9) | H (24.6) | 49.5 | MeCN |
| F8-27 | F (25.3) | I (25.4) | 50.7 | MeCN |
| F8-28 | F (25.0) | J (26.6) | 51.6 | MeCN |
| F8-29 | H (25.0) | I (25.1) | 50.1 | MeCN |
| F8-30 | F (52.3) | F (51.7) | 104.0 | acetone |
| F8-31 | F (48.7) | F (49.5) | 98.2 | EA |
| F8-32 | F (50.8) | F (49.9) | 100.7 | acetone |
| F8-33 | F (51.6) | F (52.2) | 103.8 | EA |

All examples gave a solid that was determined by XRPD to be crystalline form F.

### Example F9:

Compound (1) (crystalline form D) was dried for 122 h under vacuum (5 to 7 mbar). Details are summarized in the table below.

**Table 20:**

| **Ex.** | **m (compound (1)) [g]** | | | | | | **mass loss [wt.-%]** | **T [°C]** |
|---|---|---|---|---|---|---|---|---|
| | weighed in | after 2.25 h | after 3 h | after 4 h | after 5 h | after 122 h | | |
| F9-1 | 1.21512 | 1.17772 | 1.15760 | 1.14089 | 1.14067 | 1.04062 | 14.4 | 40 |
| F9-2 | 1.24530 | 1.06839 | 1.06753 | 1.06681 | 1.06472 | 1.06275 | 14.5 | 80 |

Both examples gave a solid that was determined by XRPD to be crystalline form F.

### Example F10:

Vials were charged with compound (1) (crystalline form D) and solvent (10 mL). The mixtures were vortexed in an Eppendorf Thermomixer for 120 h at 30°C and 400 rpm, then cooled down to 22°C and kept for 24 h without vortexing at RT. Then, the solids were separated by filtration and dried in air. Details are summarized in the table below.

**Table 21:**

| **Ex.** | **compound (1)** | | **solvent** |
|---|---|---|---|
| | m weighed in [g] | yield [wt.-%] | |
| F10-1 | 1.00380 | 57.7 | acetone |
| F10-2 | 1.03151 | 66.6 | EA |

Both examples gave a solid that was determined by XRPD to be crystalline form F.

### Example F11:

Vials were charged with compound (1) (crystalline form F) and solvent (0.5 mL). The mixtures were vortexed in an Eppendorf Thermomixer for 17 h at 23°C and 1000 rpm. Afterwards, the mixtures were kept for 3 h without vortexing at RT. Then, the solids were separated by filtration (G4 suction filter) and dried in air. Details are summarized in the table below.

**Table 22:**

| **Ex.** | **compound (1)** | **solvent** |
|---|---|---|
| | m weighed in [mg] | |
| F11-1 | 149.13 | 1PrOH |
| F11-2 | 152.23 | 1BuOH |

Both examples gave a solid that was determined by XRPD to be crystalline form F.

### Example F12:

Compound (1) (crystalline form F, F11-1, respectively F11-2) was dried in a vacuum drying cabinet. Details are summarized in the table below.

**Table 23:**

| **Ex.** | **compound (1)** | **T[°C]** | **p [mbar]** | **t[h]** | **mass loss [wt.-%]** |
|---|---|---|---|---|---|
| F12-1 | F11-1 | 40 | 50-52 | 3 | 1.5 |
| F12-2 | F11-1 | 80 | 50-52 | 3 | 2.0 |
| F12-3 | F11-1 | 115 | 5-10 | 2 | 1.3 |
| F12-4 | F11-2 | 40 | 50-52 | 2 | 4.9 |
| F12-5 | F11-2 | 80 | 50-52 | 3 | 4.7 |
| F12-6 | F11-2 | 115 | 5-10 | 2 | 4.7 |

All examples gave a solid that was determined by XRPD to be crystalline form F.

Apparently, crystalline forms A, B, C, D, E, G, H, I and J convert to crystalline form F under the given reaction conditions. This indicates that crystalline form F is the thermodynamically most stable form under the given reaction conditions.

### G) Crystalline form G

### XRPD

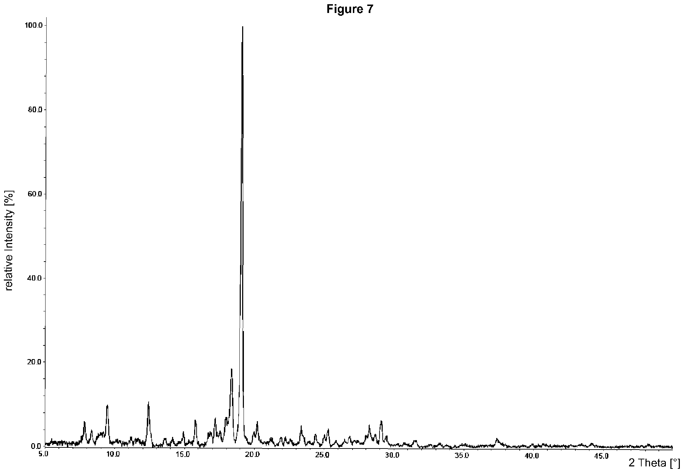

Figure 7 shows the XRPD analysis of crystalline form G.

In Table 24 below, the peak list of crystalline form G is summarized. Maximum relative intensity is 100.

**Table 24:**

| **2θ** | **rel I** | **2θ** | **rel I** | **2θ** | **rel I** |
|---|---|---|---|---|---|
| 7.8399 | 4.59 | 18.0026 | 5.86 | 26.4798 | 0.84 |
| 8.3365 | 2.74 | 18.352 | 18.31 | 26.8266 | 1.62 |
| 9.033 | 2.44 | 19.061 | 100 | 28.0037 | 1.63 |
| 9.4784 | 8.86 | 19.9679 | 2.77 | 28.2575 | 3.82 |
| 11.1534 | 1.4 | 20.2138 | 5.19 | 28.6501 | 1.96 |
| 11.65 | 0.98 | 20.9547 | 1.17 | 29.0869 | 5.5 |
| 12.4329 | 9.96 | 21.9096 | 1.94 | 29.4484 | 1.83 |
| 13.6121 | 1.51 | 22.2317 | 1.72 | 31.5123 | 1.21 |
| 14.1457 | 1.55 | 22.5937 | 1.1 | 37.4584 | 1.46 |
| 14.91 | 3.02 | 23.3896 | 3.8 | 39.9046 | 0.6 |
| 15.7925 | 5.69 | 23.941 | 1.06 | 43.407 | 0.48 |
| 16.7043 | 2.34 | 24.4017 | 2.75 | 44.1872 | 0.66 |
| 16.8819 | 2.75 | 25.0189 | 2.33 | 48.2435 | 0.4 |
| 17.2063 | 5.67 | 25.2753 | 3.71 | | |
| 17.5286 | 2.81 | 25.8275 | 0.88 | | |

### Synthesis of crystalline form G

### Example G1:

Vials were charged with compound (1) (crystalline form C, 30.7 mg) and 2PrOH (4 mL). The mixtures were vortexed using a PLS Synthesizer for 1.5 h at 50°C and 400 rpm. Afterwards, the mixtures were left over night at 30°C without shaking before the respective solvent was evaporated with pressured air. The solid was determined by XRPD to be crystalline form G.

### Example G2:

A vial was charged with compound (1) (crystalline form C, 54.0 mg) and 2PrOH (3 mL). The mixture was vortexed in an Eppendorf Thermomixer for 18 h at 30°C and 700 rpm. Afterwards, the solid was separated by filtration and dried in air. The solid was determined by XRPD to be crystalline form G.

### Example G3:

A vial was charged with compound (1) (crystalline form J, 101 mg) and 2PrOH (5 mL). The mixture was vortexed in an Eppendorf Thermomixer for 16 h at 30°C and 700 rpm. Afterwards, the mixture was stirred for 1 h at 21°C. The solid was separated and dried using a suction filter (5 sec) and was determined by XRPD to be crystalline form G.

### Example G4:

A vial was charged with compound (1) (crystalline form C, 200 mg) and 2PrOH (4.5 mL). The mixture was vortexed in an Eppendorf Thermomixer for 72 h at 25°C and 400 rpm. Afterwards, the mixture was filtered using a suction filter and the solid was dried by further suction of air through the filter cake for app. 5 min. The solid was determined by XRPD to be crystalline form G.

### H) Crystalline form H

### XRPD

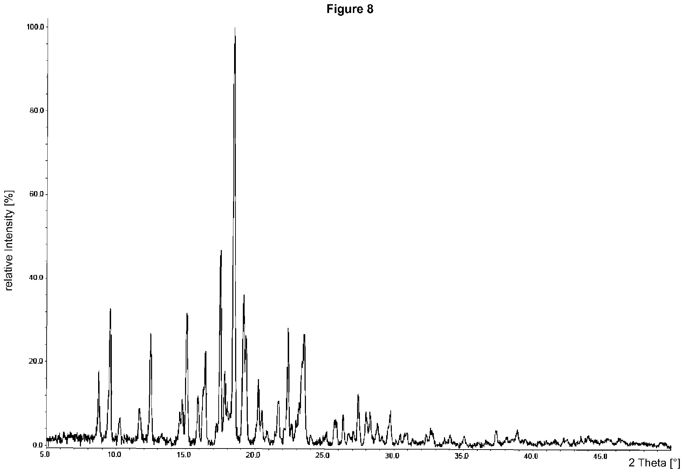

Figure 8 shows the XRPD analysis of crystalline form H.

In Table 25 below, the peak list of crystalline form H is summarized. Maximum relative intensity is 100.

**Table 25:**

| **2θ** | **rel I** | **2θ** | **rel I** | **2θ** | **rel I** |
|---|---|---|---|---|---|
| 7.3353 | 1.2 | 18.4751 | 100 | 26.7829 | 2.32 |
| 8.7547 | 15.6 | 19.1726 | 34.14 | 27.1169 | 2.65 |
| 9.5549 | 31.82 | 19.362 | 25.36 | 27.4517 | 11.5 |
| 10.2609 | 4.99 | 20.249 | 13.51 | 28.0145 | 6.99 |
| 11.7091 | 7.53 | 20.5074 | 7.43 | 28.2962 | 6.94 |
| 12.4977 | 25.58 | 20.8712 | 2.76 | 28.8367 | 4.3 |
| 13.2865 | 1.52 | 21.6787 | 10.34 | 29.7206 | 6.85 |
| 14.5968 | 6.63 | 22.1698 | 2.87 | 30.4631 | 2.15 |
| 14.7815 | 9.56 | 22.3821 | 27.51 | 30.8679 | 2.53 |
| 15.0928 | 31.52 | 22.6441 | 3.94 | 32.3433 | 1.97 |
| 15.9096 | 11.48 | 22.9621 | 3.71 | 32.6973 | 3.08 |
| 16.2919 | 12.52 | 23.168 | 8.21 | 33.6705 | 1.38 |
| 16.4371 | 21.73 | 23.3633 | 15.67 | 34.0564 | 2.1 |
| 17.2522 | 4.34 | 23.5346 | 25.59 | 35.0736 | 2.27 |
| 17.5102 | 46.23 | 24.0287 | 1.97 | 37.3733 | 3.5 |
| 17.8249 | 16.36 | 25.0958 | 2.4 | 38.1183 | 1.45 |
| 18.017 | 7.65 | 25.7959 | 6.03 | 38.8465 | 1.88 |
| 18.1973 | 4.48 | 26.3715 | 6.79 | 44.6533 | 1.37 |

### Synthesis of crystalline form H

### Example H1:

A vial was charged with compound (1) (crystalline form C) and solvent. The mixture was vortexed in an Eppendorf Thermomixer for 1.5 h at 50°C and 400 rpm. Afterwards, the solid was stored in the freezer at -18°C for 120 h. Then, when the mixture had gained RT, the solid was separated by filtration and dried in air. Details are summarized in the table below.

**Table 26:**

| **Ex.** | **solvent** | | **compound (1)** |
|---|---|---|---|
| | type | V [mL] | m (weighed in) [mg] |
| H1-1 | DCM | 1 | 30.6 |
| H1-2 | 1PrOH | 3 | 30.5 |

Both examples gave a solid that was determined by XRPD to be crystalline form H.

### Example H2:

Vials were charged with compound (1) (crystalline form C) and solvent. The mixtures were vortexed using a PLS Synthesizer for 1.5 h at 50°C and 400 rpm. Afterwards, the mixtures were left over night at 30°C without shaking before the respective solvent was evaporated with pressured air. In Table 27 below, details are summarized.

**Table 27:**

| **Ex.** | **solvent** | | **compound (1)** |
|---|---|---|---|
| | type | V [mL] | m (weighed in) [mg] |
| H2-1 | hexane | 20 | 31.6 |
| H2-2 | pentane | 20 | 31.6 |
| H2-3 | toluene | 3.5 | 30.5 |
| H2-4 | Et₂O | 4 | 31.8 |

All examples gave a solid that was determined by XRPD to be crystalline form H.

### Example H3:

A vial was charged with compound (1) (crystalline form J, 101 mg) and 1PrOH (5 mL). The mixture was vortexed in an Eppendorf Thermomixer for 16 h at 30°C and 700 rpm. Afterwards, the mixture was stirred for 1 h at 21°C. The solid was separated and dried using a suction filter (5 sec) and was determined by XRPD to be crystalline form H.

### Example H4:

A vial was charged with compound (1) (crystalline form C, 113.1 mg) and MTBE (2 mL). The mixture was vortexed in an Eppendorf Thermomixer for 68 h at 40°C and 700 rpm. Afterwards, the solid was separated using a suction filter, dried in air and was determined by XRPD to be crystalline form H.

### Example H5:

Vials were charged with compound (1) and MeCN (1 mL). The mixtures were vortexed in an Eppendorf Thermomixer for 113 h at 40°C and 1000 rpm, then cooled down to 20°C and vortexed for 2 h at 20 °C and 1000 rpm. Then, the solids were separated by filtration and dried in air. Details are summarized in the table below.

**Table 28:**

| **Ex.** | **compound (1)** | | |
|---|---|---|---|
| | crystalline form (m [mg]) | crystalline form (m [mg]) | Cₜₒₜₐₗ [mg/mL] |
| H5-1 | H (25.8) | J (23.9) | 49.7 |
| H5-2 | I (24.8) | J (23.2) | 48.0 |

Both examples gave a solid that was determined by XRPD to be crystalline form H.

### Example H6:

Compound (1) was dried in a drying cabinet at 80°C and 5 to 7 mbar. Details are summarized in the table below.

**Table 29:**

| **Ex.** | **crystalline form** | **t [h]** |
|---|---|---|
| H6-1 | K | 40 |
| H6-2 | H | 17 |

Both examples gave a solid that was determined by XRPD to be crystalline form H.

### I) Crystalline form I

### XRPD

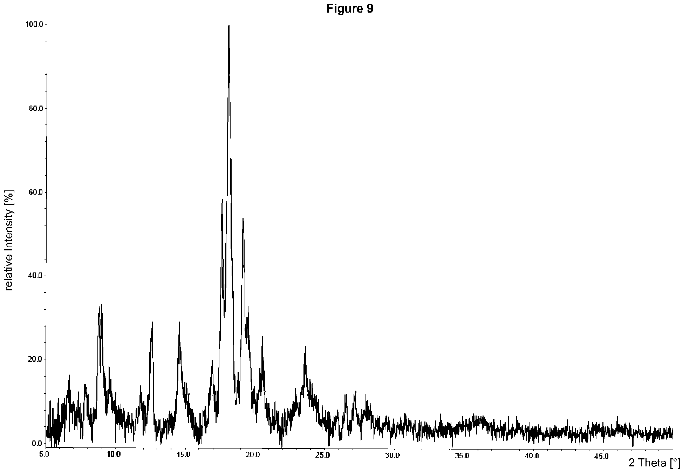

Figure 9 shows the XRPD analysis of crystalline form I.

In Table 30 below, the peak list of crystalline form I is summarized. Maximum relative intensity is 100.

**Table 30:**

| **2θ** | **rel I** | **2θ** | **rel I** |
|---|---|---|---|
| 6.6412 | 8.27 | 18.0701 | 100 |
| 7.8712 | 9.59 | 18.3661 | 19.11 |
| 8.7969 | 25.38 | 19.1266 | 48.81 |
| 9.0246 | 24.02 | 19.5001 | 25.06 |
| 9.6176 | 7.8 | 20.5244 | 18.06 |
| 11.8072 | 7.92 | 23.648 | 12.72 |
| 12.5774 | 27.53 | 26.5309 | 7.93 |
| 14.6421 | 21.69 | 27.1579 | 8.59 |
| 16.8842 | 15.36 | 28.0399 | 7.36 |
| 17.5954 | 50.49 | | |

### Synthesis of crystalline form I

### Example 11:

A vial was charged with compound (1) (crystalline form C, 31 mg) and 2PrOH (10 mL). The mixture was vortexed using a PLS Synthesizer for 3 h at 40°C and 400 rpm. Afterwards, the mixtures were left for 3 d at 23°C without shaking. Then, the solvents were evaporated with pressured air at 40°C. The obtained solid was determined by XRPD to be crystalline form I.

### Example 12:

Compound (1) (crystalline form I) was stored for 48 h at -40°C. Afterwards, it was determined by XRPD that the crystalline form had been retained (crystalline form I).

### Example 13:

Compound (1) was stored in a drying cabinet at 115°C and 50 mbar. Details are summarized in the table below.

**Table 31:**

| **Ex.** | **compound (1) crystalline form (starting material)** | **t [h]** |
|---|---|---|
| I3-1 | A (Ex. A4) | 2 |
| I3-2 | A (Ex. A4) | 17 |
| I3-3 | E (Ex. E5) | 2 |
| I3-4 | E (Ex. E5) | 17 |
| I3-5 | G (Ex. G4) | 2 |
| I3-6 | G (Ex. G4) | 17 |
| I3-7 | C (Ex. C4) | 2 |
| I3-8 | C (Ex. C4) | 17 |

All examples gave a solid that was determined by XRPD to be crystalline form I.

### J) Crystalline form J

### XRPD

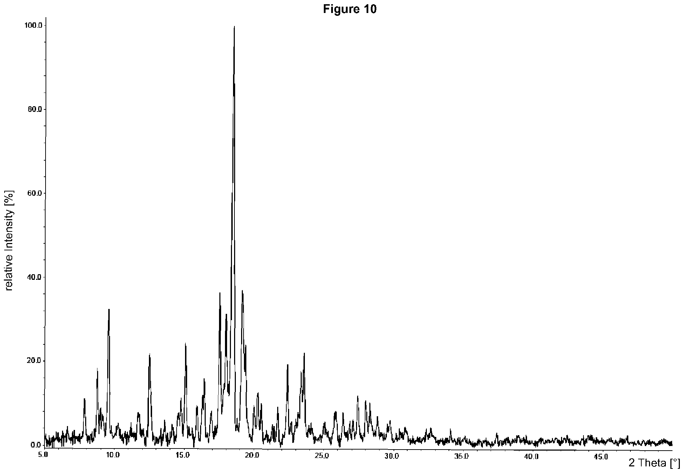

Figure 10 shows the XRPD analysis of crystalline form J.

In Table 32 below, the peak list of crystalline form J is summarized. Maximum relative intensity is 100.

**Table 32:**

| **2θ** | **rel I** | **2θ** | **rel I** | **2θ** | **rel I** |
|---|---|---|---|---|---|
| 7.8757 | 9.92 | 16.4428 | 15.11 | 23.3737 | 15.39 |
| 8.7722 | 16.14 | 16.9374 | 7.13 | 23.5795 | 21.76 |
| 8.9817 | 6.77 | 17.5367 | 35.33 | 25.068 | 3.2 |
| 9.1625 | 5.21 | 17.9925 | 23.81 | 25.8394 | 6.96 |
| 9.5649 | 32.05 | 18.4668 | 100 | 26.4026 | 5.68 |
| 10.2459 | 3.45 | 19.1619 | 37.58 | 26.8696 | 4.24 |
| 11.1844 | 3.03 | 19.3806 | 20.26 | 27.1159 | 4.24 |
| 11.7367 | 6.74 | 20.0194 | 8.86 | 27.4595 | 10.9 |
| 12.5346 | 21.66 | 20.2878 | 12.87 | 28.0194 | 9.5 |
| 24.0478 | 3.26 | 20.5317 | 10.21 | 28.3441 | 6.33 |
| 13.6145 | 3.85 | 20.9011 | 2.36 | 28.8592 | 4.03 |
| 14.1587 | 3.7 | 21.3169 | 4.24 | 29.7199 | 3.95 |
| 14.5832 | 6.58 | 21.7047 | 8.77 | 30.8668 | 2.31 |
| 14.7857 | 8.27 | 22.3923 | 17.93 | 34.0897 | 2.49 |
| 15.1031 | 23.91 | 22.6645 | 4.94 | 37.4084 | 2.33 |
| 15.922 | 9.13 | 22.9932 | 4.53 | | |
| 16.3023 | 9.34 | 23.1759 | 6.14 | | |

### Synthesis of crystalline form J

### Example J1:

Vials were charged with compound (1) (crystalline form C) and solvent. The mixtures were vortexed using a PLS Synthesizer for 1.5 h at 50°C and 400 rpm. Afterwards, Ex. J1-1 to J1-3 were stored for 120 h in a freezer at -18°C before the respective solvent was evaporated with pressured air at 23°C. In case of Ex. J1-4, the mixtures were left over night at 30°C without shaking before the respective solvent was evaporated with pressured air. In Table 33 below, details are summarized.

**Table 33:**

| **Ex.** | **solvent** | | **compound (1)** |
|---|---|---|---|
| | type | V [mL] | m (weighed in) [mg] |
| J1-1 | toluene | 3 | 31.0 |
| J1-2 | MEK | 0.5 | 31.9 |
| J1-3 | MeCN | 10 | 31.3 |
| J1-4 | MeCN | 10 | 31.4 |

All examples gave a solid that was determined by XRPD to be crystalline form J.

### Example J2:

A vial was charged with compound (1) (crystalline form H, 112.2 mg) and MeCN (15 mL). The solution was vortexed using a PLS Synthesizer for 24 h at 23°C and 400 rpm. Then, the solvent was evaporated with pressured air. The solid was separated by filtration and dried by suction of air through the filter cake for app. 1 h. The solid was determined by XRPD to be crystalline form J.

### Example J3:

Compound (1) (crystalline form J) was stored for 48 h at -40°C. Afterwards, it was determined by XRPD that the crystalline form had been retained (i.e. crystalline form J).

### Example J4:

Compound (1) (crystalline form E) was dried in a drying cabinet for 17 h at 23°C and ambient pressure. The obtained solid was determined by XRPD to be crystalline form J.

### K) Crystalline form K

### XRPD

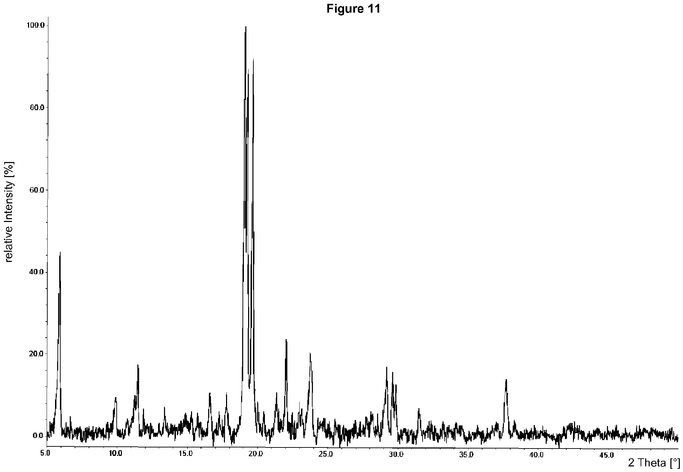

Figure 11 shows the XRPD analysis of crystalline form K.

In Table 34 below, the peak list of crystalline form K is summarized. Maximum relative intensity is 100.

**Table 34:**

| **2θ** | **rel I** | **2θ** | **rel I** | **2θ** | **rel I** |
|---|---|---|---|---|---|
| 5.8601 | 43.29 | 17.2827 | 4.47 | 23.0388 | 4.87 |
| 9.8601 | 8.9 | 17.801 | 7.92 | 23.7639 | 18.89 |
| 11.2817 | 6.92 | 19.0461 | 100 | 20.7519 | 0.44 |
| 11.4671 | 13.67 | 19.239 | 84.5 | 28.0751 | 2.76 |
| 13.3919 | 5.44 | 19.6144 | 92.85 | 29.1725 | 14.54 |
| 14.8764 | 2.88 | 20.04 | 6.71 | 29.6805 | 10.35 |
| 15.3056 | 4.08 | 20.4402 | 5.34 | 37.7023 | 12.43 |
| 15.7284 | 3.4 | 21.3566 | 8.12 | | |
| 16.6172 | 9.95 | 22.0271 | 23.68 | | |

### Synthesis of crystalline form K

### Example K1:

The synthesis of the hemi citrate salt of compound (1) is disclosed in WO 2004/043967 (Example 49).

At 40°C, hemi citrate salt of compound (1) (49.74 mg) was dissolved in DMSO (2.5 mL). Glucose solution (5 wt.-%, 3 mL) was added and the resulting suspension was vortexed using a PLS Synthesizer for 3 d at 40°C and 400 rpm. After 6 d and cooling to RT, the solid is separated by filtration and dried by suction of air through the filter cake. The obtained solid was determined by XRPD to be crystalline form K.

### X) Further Analyses of the crystalline forms A to K

### Differential Scanning Calorimetry (DSC)

The measurement was realized using a Mettler Toledo DSC821 or Mettler Toledo DSC823. Unless otherwise specified, the samples were weighed in a pierced aluminium crucible. The measurement took place in a nitrogen flow in a temperature range from -50°C up to 350°C with a heating rate of 10°C/min. The temperatures specified in relation to DSC analyses are, unless otherwise specified, the temperatures of the peak onset.

In the following table, the results of one or more typical DSC thermograms of the crystalline forms are summarized. "ΔH" means "specific heat", "Tₒₙₛₑₜ" means the "onset temperature", and "Tₚₑₐₖ" means the "peak temperature" of a thermal event. Some crystalline forms showed more than one thermal event, in those cases ΔH, Tₒₙₛₑₜ and Tₚₑₐₖ are listed for each event.

**Table 35:**

| **crystalline form** | **Tₒₙₛₑₜ [°C]** | **Tₚₑₐₖ [°C]** | **ΔH [J/g]** |
|---|---|---|---|
| A | 81.8 | 100.8 | 55.6 |
| | 117.8 | 117.9 | 91.0 |
| | 257.0 | 260.3 | 128.0 |
| | 82.5 | 92.1 | 2.4 |
| | 99.6 | 126.8 | 43.6 |
| | 257.8 | 260.5 | 109.2 |
| B | 186.3 | 192.5 | 2.2 |
| | 246.1 | 251.1 | 38.8 |
| | 255.2 | 256.75 | 71.6 |
| C | 156.5 | 156.7 | 63.2 |
| | 187.4 | 191.3 | 1.8 |
| | 253.9 | 255.0 | 119.0 |
| | 191.3 | 207.2 | 1.4 |
| | 254.5 | 255.0 | 122.6 |
| D | 75.9 | 93.7 | 66.1 |
| | 245.2 | 257.9 | 28.0 |
| | 258.1 | 261.9 | 81.0 |
| | 79.3 | 87.2 | 142.8 |
| | 193.7 | 201.6 | 11.4 |
| | 255.6 | 256.0 | 107.6 |
| E | 87.5 | 95.5 | 128.9 |
| | 252.5 | 253.7 | 111.5 |
| | 86.9 | 97.8 | 131.9 |
| | 253.7 | 256.1 | 104.9 |
| | 88.9 | 99.1 | 139.9 |
| | 191.4 | 201.7 | 3.2 |
| | 254.5 | 256.0 | 102.4 |
| F | 193.6 | 200.5 | 11.6 |
| | 254.4 | 255.4 | 123.3 |
| | 204.4 | 208.5 | 11.1 |
| | 255.5 | 256.4 | 120.1 |
| G | 81.31 | 93.80 | 123.81 |
| | 254.56 | 255.48 | 105.79 |
| | 90.68 | 105.04 | 140.85 |
| | 210.53 | 218.55 | -2.00 |
| | 254.64 | 255.83 | 101.81 |
| | 90.45 | 104.98 | 141.96 |
| | 210.96 | 217.86 | -1.88 |
| | 254.48 | 256.13 | 103.96 |
| H | 253.60 | 257.17 | 121.94 |
| I | 182.23 | 193.75 | -5.37 |
| | 254.29 | 255.19 | 122.53 |
| J | 254.16 | 254.83 | 118.59 |
| K | no data available | | |

### Thermogravimetry analysis (TGA)

TGA experiments were recorded with a Mettler Toledo TGA/DSC1 (open aluminium oxide crucible nitrogen atmosphere, heating rate 10°C/min, 25 up to 350°C). The results are summarized in the table below.

**Table 36:**

| **crystalline form** | **typical weight loss in wt.-%** |
|---|---|
| A | 7.0 |
| | 7.3 |
| B | 0.5 |
| C | 0 |
| D | 5.5 |
| | 13.8 |
| E | 10.7 |
| | 8.4 |
| | 9.9 |
| F | 0.1 |
| | 0.1 |
| | 0.4 |
| G | 9.0 |
| | 12.6 |
| | 11.8 |
| H | 0 |
| I | 0.1 |
| J | 0 |
| K | not measured |

According to the results summarized in the table above, it can be assumed that crystalline forms B, C, F, H, I and J are ansolvate forms.

### SCXRD (Single Crystal X-ray Diffraction)

SCXRD analyses of crystalline forms was carried out with a Bruker D8-goniometer with SMART APEX CCD area detector at 100 K (± 5 K) using MοK_{α} radiation (wavelength of 0.71073 Å, Incoatec microsource, multilayer optics). The results are summarized in the table below. Values given in parentheses signify standard deviations.

**Table 37:**

| **crystalline form** | **B** | **F** |
|---|---|---|
| **a/Å** | 14.2570(13) | 10.271(4) |
| **b/Å** | 13.7195(13) | 16.370(5) |
| **c/Å** | 19.3867(18) | 11.2612(12) |
| **α/°** | | |
| **β/°** | 100.529(3) | 100.70(2) |
| **γ/°** | | |
| **volume/ Å**³ | 3728.2(6) | 1860.4(9) |
| **space group** | P2₁/c | P2₁/c |
| **temp. measured/K** | 110(2) | 238(2) |

### Variable temperature x-ray powder diffraction (VTXRPD)

The VTXRPD analysis of crystalline forms was performed in a temperature range from 25°C to 210°C. A heating rate of 10°C/min was applied and hold-time per step was 15 min (if not indicated otherwise). Details are summarized in the table below.

**Table 38:**

| **crystalline form (start)** | **transition temperature** | **crystalline form (end)** | **comments** |
|---|---|---|---|
| H | no transition | H | melts at 250°C |
| F | 180-190°C | H | melts at 250°C |
| C (batch 1) | 200-210°C | H | melts at 250°C, 15 min per temperature step |
| C (batch 1) | no transition | C | melts at 220°C, 30 min per temperature step |
| C (batch 2) | no transition | C | melts at 240°C, 15 min per temperature step |
| C (batch 2) | no transition | C | melts at 240°C, 30 min per temperature step |
| C (batch 3) | 200-210°C | H | melts at 250°C |

### Dynamic vapor sorption (DVS)

Crystalline forms D and F were analyzed via DVS using a Porotec DVS at 25°C. A step width of 10 % r.h. was applied allowing the sample to equilibrate and reach weight constancy (± 0.002 %) for at least 10 min on each step. The measurement was started with sorption from 50-90% rH followed by a full cycle 90-0-90% rH and finished by desorption 90-50% rH. The hygroscopicity determined via the DVS measurements was classified according to the ranges for mass increase defined in the European Pharmacopoeia: very hygroscopic (vh): increase of the mass ≥ 15 %; hygroscopic (h): increase of the mass is less than 15 % and equal or greater than 2 %; slightly hygroscopic (sh): increase of the mass is less than 2 % and equal or greater than 0.2 %; not hygroscopic (nh): increase of the mass is less than 0.2 %; deliquescent (d): sufficient water is absorbed to form a liquid.

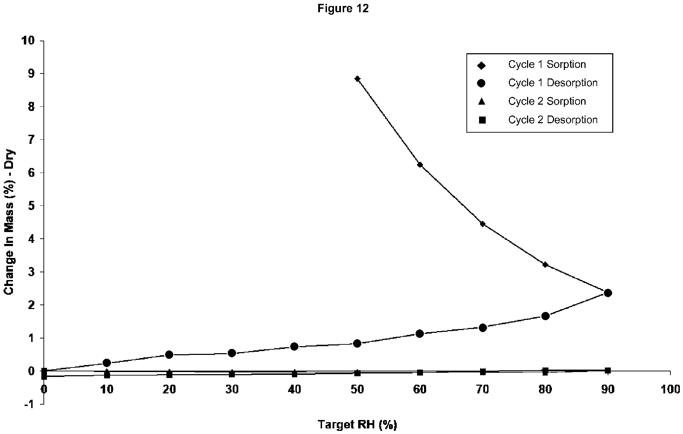

The DVS isotherm plot of crystalline form D is shown in Figure 12. The data from the first sorption cycle suggest that the solvent molecule (2PrOH) is replaced by water. Further lowering of ambient humidity shows that the water is removed almost completely resulting in a form that is no longer hygroscopic. This can be derived from the second sorption/desorption cycle that does not exhibit any change in mass >0.1%.

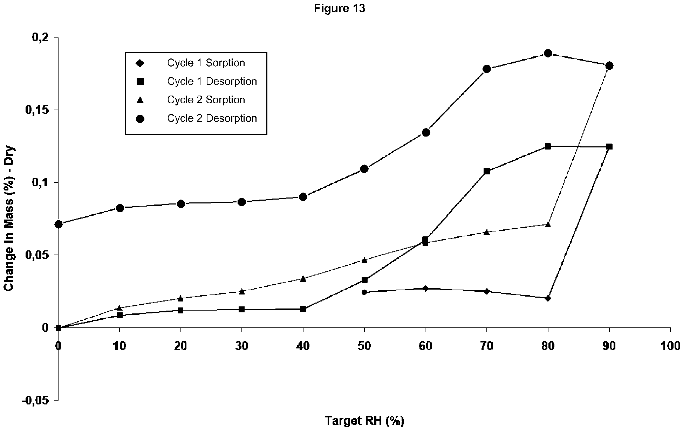

The DVS isotherm plot of crystalline form F is shown in Figure 13. From the data it can be seen that there is only a very slight water sorption up to a maximum mass change of 0.2% occurring at rH above 80%. Desorption shows a hysteresis down to about 40% rH. Based on this, crystalline form F can be considered as non-hygroscopic.

## Claims

1. A crystalline form of the compound according to formula (I) 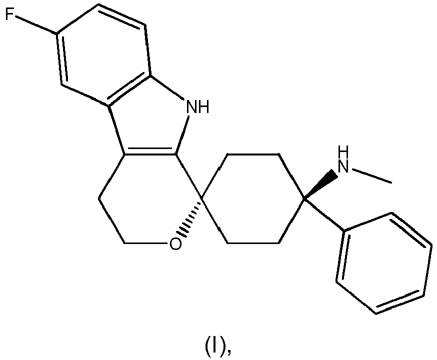 having at least one X-ray powder diffraction peak (CuK_{α} radiation) in the range of from 9.3±0.2 to 11.3±0.2 (2θ) and/or 16.0±0.2 to 18.0±0.2 (2θ) and/or 17.0±0.2 to 19.5±0.2 (2θ) and/or 18.5±0.2 to 21.0±0.2 (2θ).

2. The crystalline form according to claim 1, which is an ansolvate or a solvate.

3. The crystalline form according to claim 2, which is a solvate selected from the group consisting of methanol solvate, 2-propanol solvate, ethanol solvate and dimethyl sulfoxide solvate.

4. The crystalline form according to any of the preceding claims, which has at least one additional X-ray powder diffraction peak (CuK_{α} radiation) in the range of from 23.7±0.2 to 25.7±0.2 (2θ) and/or 19.9±0.2 to 21.9±0.2 (2θ) and/or 17.4±0.2 to 19.4±0.2 (2θ).

5. The crystalline form according to any of the preceding claims, which exhibits in differential scanning calorimetry analysis
at least one endothermic event with an onset temperature in the range of from 70°C to 90°C and/or 80°C to 100°C and/or 90°C to 110°C and/or 180°C to 215°C and/or 240°C to 270°C; and/or
a peak temperature in the range of from 80°C to 100°C and/or 90°C to 110°C and/or 105°C to 130°C and/or 190°C to 220°C and/or 245°C to 275°C.

6. The crystalline form according to any of the preceding claims, which is
crystalline form A having at least one X-ray powder diffraction peak (CuK_{α} radiation) selected from the group consisting of 19.5±0.2 (2θ), 14.2±0.2 (2θ), 9.9±0.2 (2θ), 10.3±0.2 (2θ), 17.6±0.2 (2θ), 26.0±0.2 (2θ), 17.2±0.2 (2θ), 15.8±0.2 (2θ) and 22.4±0.2 (2θ); or
crystalline form B having at least one X-ray powder diffraction peak (CuK_{α} radiation) selected from the group consisting of 18.4±0.2 (2θ), 19.1±0.2 (2θ), 7.9±0.2 (2θ), 18.0±0.2 (2θ), 20.0±0.2 (2θ), 12.6±0.2 (2θ), 16.9±0.2 (2θ) and 9.0±0.2 (2θ); or
crystalline form C having at least one X-ray powder diffraction peak CuK_{α} radiation) selected from the group consisting of 18.1±0.2 (2θ), 17.6±0.2 (2θ), 19.1±0.2 (2θ), 8.8±0.2 (2θ), 12.6±0.2 (2θ), 19.5±0.2 (2θ), 14.6±0.2 (2θ), 16.9±0.2 (2θ) and 20.5±0.2 (2θ); or
crystalline form D having at least one X-ray powder diffraction peak (CuK_{α} radiation) selected from the group consisting of 19.4±0.2 (2θ), 12.0±0.2 (2θ), 20.7±0.2 (2θ), 16.5±0.2 (2θ), 11.7±0.2 (2θ), 10.4±0.2 (2θ), 14.5±0.2 (2θ) and 16.8±0.2 (2θ); or
crystalline form E having at least one X-ray powder diffraction peak (CuK_{α} radiation) selected from the group consisting of 10.3±0.2 (2θ), 18.4±0.2 (2θ), 22.6±0.2 (2θ), 20.6±0.2 (2θ), 9.2±0.2 (2θ), 13.8±0.2 (2θ), 17.8±0.2 (2θ), 17.3±0.2 (2θ), 18.9±0.2 (2θ), 23.4±0.2 (2θ), 18.6±0.2 (2θ), 19.4±0.2 (2θ), 21.4±0.2 (2θ), 24.9±0.2 (2θ) and 19.1±0.2 (2θ); or
crystalline form F having at least one X-ray powder diffraction peak (CuK_{α} radiation) selected from the group consisting of 16.9±0.2 (2θ), 16.0±0.2 (2θ), 24.7±0.2 (2θ), 20.9±0.2 (2θ), 18.4±0.2 (2θ), 25.6±0.2 (2θ) and 17.6±0.2 (2θ); or
crystalline form G having at least one X-ray powder diffraction peak (CuK_{α} radiation) selected from the group consisting of 19.1±0.2 (2θ), 18.4±0.2 (2θ), 12.4±0.2 (2θ), 9.5±0.2 (2θ), 18.0±0.2 (2θ), 15.8±0.2 (2θ), 17.2±0.2 (2θ), 29.1±0.2 (2θ) and 20.2±0.2 (2θ); or
crystalline form H having at least one X-ray powder diffraction peak (CuK_{α} radiation) selected from the group consisting of 18.5±0.2 (2θ), 17.5±0.2 (2θ), 19.2±0.2 (2θ), 9.6±0.2 (2θ), 15.1±0.2 (2θ), 22.4±0.2 (2θ), 23.5±0.2 (2θ), 12.5±0.2 (2θ), 19.4±0.2 (2θ) and 16.4±0.2 (2θ); or
crystalline form I having at least one X-ray powder diffraction peak (CuK_{α} radiation) selected from the group consisting of 18.1±0.2 (2θ), 17.6±0.2 (2θ), 19.1±0.2 (2θ), 12.6±0.2 (2θ), 8.8±0.2 (2θ), 19.5±0.2 (2θ), 9.0±0.2 (2θ) and 14.6±0.2 (2θ); or
crystalline form J having at least one X-ray powder diffraction peak (CuK_{α} radiation) selected from the group consisting of 18.5±0.2 (2θ), 19.2±0.2 (2θ), 17.5±0.2 (2θ), 9.6±0.2 (2θ), 15.1±0.2 (2θ), 18.0±0.2 (2θ), 23.6±0.2 (2θ), 12.5±0.2 (2θ) and 19.4±0.2 (2θ); or
crystalline form K having at least one X-ray powder diffraction peak (CuK_{α} radiation) selected from the group consisting of 19.0±0.2 (2θ), 19.6±0.2 (2θ), 19.2±0.2 (2θ), 5.9±0.2 (2θ), 22.0±0.2 (2θ), 23.8±0.2 (2θ), 29.2±0.2 (2θ), 11.5±0.2 (2θ), 37.7±0.2 (2θ) and 29.7±0.2 (2θ).

7. The crystalline form according to any of the preceding claims, which is
crystalline form A exhibiting in differential scanning calorimetry analysis a first endothermic event with an onset temperature in the range of from 70°C to 90°C and/or a peak temperature in the range of from 80°C to 110°C; a second endothermic event with an onset temperature in the range of from 80°C to 125°C and/or a peak temperature in the range of from 105°C to 135°C; and a third endothermic event with an onset temperature in the range of from 245°C to 265°C and/or a peak temperature in the range of from 250°C to 270°C; or
crystalline form B exhibiting in differential scanning calorimetry analysis a first endothermic event with an onset temperature in the range of from 175°C to 195°C and/or a peak temperature in the range of from 180°C to 200°C; a second endothermic event with an onset temperature in the range of from 235°C to 255°C and/or a peak temperature in the range of from 240°C to 260°C; and a third endothermic event with an onset temperature in the range of from 245°C to 265°C and/or a peak temperature in the range of from 247°C to 267°C; or
crystalline form C exhibiting in differential scanning calorimetry analysis a first endothermic event with an onset temperature in the range of from 177°C to 200°C and/or a peak temperature in the range of from 180°C to 217°C; a second endothermic event with an onset temperature in the range of from 243°C to 263°C and/or a peak temperature in the range of from 245°C to 265°C; or
crystalline form D exhibiting in differential scanning calorimetry analysis a first endothermic event with an onset temperature in the range of from 65°C to 90°C and/or a peak temperature in the range of from 77°C to 104°C; a second endothermic event with an onset temperature in the range of from 180°C to 200°C or 235°C to 255°C and/or a peak temperature in the range of from 190°C to 210°C or 248°C to 268°C; and a third endothermic event with an onset temperature in the range of from 245°C to 265°C and/or a peak temperature in the range of from 247°C to 267°C; or
crystalline form E exhibiting in differential scanning calorimetry analysis a first endothermic event with an onset temperature in the range of from 77°C to 99°C and/or a peak temperature in the range of from 86°C to 110°C; and a second endothermic event with an onset temperature in the range of from 243°C to 265°C and/or a peak temperature in the range of from 245°C to 267°C; or
crystalline form F exhibiting in differential scanning calorimetry analysis a first endothermic event with an onset temperature in the range of from 180°C to 215°C and/or a peak temperature in the range of from 190°C to 220°C; and a second endothermic event with an onset temperature in the range of from 240°C to 270°C and/or a peak temperature in the range of from 245°C to 275°C; or
crystalline form G exhibiting in differential scanning calorimetry analysis a first endothermic event with an onset temperature in the range of from 70°C to 90°C or 80°C to 100°C and/or a peak temperature in the range of from 84°C to 104°C or 95°C to 115°C; an optional exothermic event with an onset temperature in the range of from 200°C to 220°C and/or a peak temperature in the range of from 208°C to 230°C; and a second endothermic event with an onset temperature in the range of from 244°C to 265°C and/or a peak temperature in the range of from 245°C to 266°C; or
crystalline form H exhibiting in differential scanning calorimetry analysis an endothermic event with an onset temperature in the range of from 244°C to 264°C and/or a peak temperature in the range of from 247°C to 267°C; or
crystalline form I exhibiting in differential scanning calorimetry analysis an exothermic event with an onset temperature in the range of from 172°C to 192°C and/or a peak temperature in the range of from 184°C to 204°C; and an endothermic event with an onset temperature in the range of from 244°C to 264°C and/or a peak temperature in the range of from 245°C to 265°C; or
crystalline form J exhibiting in differential scanning calorimetry analysis an endothermic event with an onset temperature in the range of from 244°C to 264°C and/or a peak temperature in the range of from 245°C to 265°C.

8. The crystalline form according to any of the preceding claims, which is
crystalline form A having X-ray powder diffraction peaks (CuK_{α} radiation) at 19.5±0.2 (2θ), 14.2±0.2 (2θ) and 9.9±0.2 (2θ); or
crystalline form B having X-ray powder diffraction peaks (CuK_{α} radiation) at 18.4±0.2 (2θ), 19.1±0.2 (2θ) and 7.9±0.2 (2θ); or
crystalline form C having X-ray powder diffraction peaks (CuK_{α} radiation) at 18.1±0.2 (2θ), 17.6±0.2 (2θ) and 19.1±0.2 (2θ); or
crystalline form D having X-ray powder diffraction peaks (CuK_{α} radiation) at 19.4±0.2 (2θ), 12.0±0.2 (2θ) and 20.7±0.2 (2θ); or
crystalline form E having X-ray powder diffraction peaks (CuK_{α} radiation) at 10.3±0.2 (2θ), 18.4±0.2 (2θ) and 22.6±0.2 (2θ); or
crystalline form F having X-ray powder diffraction peaks (CuK_{α} radiation) at 16.9±0.2 (2θ), 16.0±0.2 (2θ) and 24.7±0.2 (2θ); or
crystalline form G having X-ray powder diffraction peaks (CuK_{α} radiation) at 19.1±0.2 (2θ), 18.4±0.2 (2θ) and 12.4±0.2 (2θ); or
crystalline form H having X-ray powder diffraction peaks (CuK_{α} radiation) at 18.5±0.2 (2θ), 17.5±0.2 (2θ) and 19.2±0.2 (2θ); or
crystalline form I having X-ray powder diffraction peaks (CuK_{α} radiation) at 18.1±0.2 (2θ), 17.6±0.2 (2θ) and 19.1±0.2 (2θ); or
crystalline form J having X-ray powder diffraction peaks (CuK_{α} radiation) at 18.5±0.2 (2θ), 19.2±0.2 (2θ) and 17.5±0.2 (2θ); or
crystalline form K having X-ray powder diffraction peaks (CuK_{α} radiation) at 19.0±0.2 (2θ), 19.6±0.2 (2θ) and 19.2±0.2 (2θ).

9. The crystalline form according to any of claims 6 to 8, which is crystalline form F, B, H, A, C, D, E, G, I, J or K.

10. The crystalline form according to any of the preceding claims, which is crystalline form F having X-ray powder diffraction peaks (CuK_{α} radiation) at 16.9±0.2 (2θ), 16.0±0.2 (2θ) and 24.7±0.2 (2θ); and/or
exhibiting in differential scanning calorimetry analysis a first endothermic event with an onset temperature in the range of from 180°C to 215°C and/or a peak temperature in the range of from 190°C to 220°C; and a second endothermic event with an onset temperature in the range of from 240°C to 270°C and/or a peak temperature in the range of from 245°C to 275°C.

11. The crystalline form according to any of the preceding claims, which is crystalline form B having X-ray powder diffraction peaks (CuK_{α} radiation) at 18.4±0.2 (2θ), 19.1±0.2 (2θ) and 7.9±0.2 (2θ); and/or
exhibiting in differential scanning calorimetry analysis a first endothermic event with an onset temperature in the range of from 175°C to 195°C and/or a peak temperature in the range of from 180°C to 200°C; a second endothermic event with an onset temperature in the range of from 235°C to 255°C and/or a peak temperature in the range of from 240°C to 260°C; and a third endothermic event with an onset temperature in the range of from 245°C to 265°C and/or a peak temperature in the range of from 247°C to 267°C.

12. A pharmaceutical dosage form comprising at least one crystalline form according to any of claims 1 to 11.

13. A process for preparing a crystalline form according to any of claims 1 to 11, comprising the steps of
(i) mixing the compound according to formula (I) with a solvent;
(ii) stirring at room temperature or stirring at an elevated temperature the mixture obtained in step (i);
(iii) separating the solid from the solvent; and
(iv) drying the solid obtained in step (iii).

14. The process according to claim 13, wherein the solvent used in step (i) is selected from the group consisting of ethyl acetate, acetone and acetonitrile.

15. The process according to claim 13 or 14, wherein step (ii) is realized at a temperature in the range of from 20°C to 50°C.
